# FASCICULE DE BREVET EUROPEEN

(11) **EP 1 682 787 B1**
(45) Date de publication et mention de la délivrance du brevet: **07.05.2008**
(21) Numéro de dépôt: 04805816.8
(22) Date de dépôt: 08.11.2004
(51) Int. Cl.: F16D 29/00, F15B 15/20, F15B 15/02, F16D 23/12

(54) **SYSTEME DE COMMANDE HYDRAULIQUE D'UN EMBRAYAGE COMPORTANT DES MOYENS D'ASSISTANCE INTERPOSES ENTRE L' ÉMETTEUR ET LE RECEPTEUR DU SYSTÈME.& x9;**
HYDRAULISCHES KUPPLUNGSBETÄTIGUNGSSYSTEM MIT ZWISCHEN DEM HAUPTZYLINDER UND DEM NEHMERZYLINDER DES SYSTEMS ANGEORDNETEN SERVOMITTELN
HYDRAULIC CLUTCH CONTROL SYSTEM, COMPRISING SERVO MEANS WHICH ARE DISPOSED BETWEEN THE MASTER CYLINDER AND THE SLAVE CYLINDER OF THE SYSTEM

(30) Priorité: 12.11.2003 FR 0350826
(43) Date de publication de la demande: 26.07.2006
(73) Titulaire: Valeo Embrayages, 80009 Amiens (FR)
(72) Inventeur: VILLATA, Gino, I-14021 BUTTIGLIERA D'ASTI (IT); THERY, Pascal, F-80000 AMIENS (FR)
(86) Numéro de dépôt international: PCT/FR2004/050573
(87) Numéro de publication internationale: WO 2005/047722

(56) Documents cités:
- DE-A- 19 509 356
- DE-A- 19 717 486
- GB-A- 2 236 153
- GB-A- 2 313 885
- US-A- 3 812 766
- US-A- 5 809 830

## Description

L'invention concerne un système de commande hydraulique d'un embrayage.

L'invention concerne plus particulièrement un système de commande hydraulique d'un embrayage, notamment de véhicule automobile, comprenant un cylindre émetteur amont, qui est relié par une conduite à un cylindre récepteur aval, de manière à former un circuit hydraulique de commande.

Il est parfois souhaitable d'équiper le système de commande hydraulique d'un embrayage d'un dispositif d'assistance, de manière à minimiser l'effort que l'utilisateur doit appliquer à la pédale de commande de l'embrayage, lors de la phase de débrayage.

Un tel dispositif est décrit par exemple dans le document US-B-6.213.271 .

Dans ce document, le dispositif d'assistance est monté sur le cylindre émetteur du système de commande hydraulique d'embrayage.

Ce système présente l'inconvénient de nécessiter un cylindre émetteur spécifique, adapté à l'agencement des éléments supplémentaires réalisant la fonction d'assistance.

L'agencement des éléments supplémentaires sur le cylindre émetteur pose des problèmes d'encombrement et cela rend le cylindre émetteur plus complexe à réaliser.

La présente invention vise à remédier à ces inconvénients en proposant une solution simple et économique, qui ne nécessite pas de modifier le cylindre émetteur ou le cylindre récepteur.

Dans ce but, l'invention propose un système de commande du type décrit précédemment, caractérisé en ce qu'il comporte un cylindre d'assistance qui est interposé dans la conduite, entre le cylindre émetteur et le cylindre récepteur, et qui comporte au moins un piston d'assistance qui est monté coulissant axialement dans le corps du cylindre d'assistance entre une position amont d'embrayage et une position aval de débrayage, de manière à délimiter une chambre hydraulique amont et une chambre hydraulique aval de volumes variables en fonction de la position axiale du piston, la chambre amont étant raccordée au cylindre émetteur par un tronçon du circuit hydraulique dit circuit amont et la chambre aval étant raccordée au cylindre récepteur par un tronçon du circuit hydraulique dit circuit aval, chaque tronçon de circuit hydraulique comportant un moyen de remise à niveau du volume de fluide raccordé à au moins un réservoir de fluide, et en ce que le cylindre d'assistance comporte un dispositif d'assistance qui applique une force d'assistance sur le piston d'assistance au cours de la phase de débrayage.

Le document US-A-5.809.830 et GB-A-2.236.153 décrivent et représentent des dispositifs d'assistance comportant des pistons mus électriquement.

Les documents DE-A-19.717.486 et DE-A-19.509.356 décrivent et représentent des dispositifs d'assistance comportant des pistons mus pneumatiquement.

Un avantage du système selon l'invention est qu'il utilise un cylindre émetteur et un cylindre récepteur du type standard, qui n'ont pas été prévus pour être équipés d'un dispositif d'assistance.

De plus, le système de commande d'embrayage selon l'invention peut être agencé dans un véhicule sans qu'il soit nécessaire de modifier la zone d'agencement du cylindre émetteur et/ou la zone d'agencement du cylindre récepteur, par rapport à un véhicule similaire non équipé du dispositif d'assistance, l'encombrement du cylindre émetteur et l'encombrement du cylindre récepteur n'étant pas modifiés.

Un autre avantage du système de commande selon l'invention est que le cylindre d'assistance et son dispositif d'assistance n'ont pas d'influence sur la loi de commande liant le déplacement de la pédale de commande de l'embrayage au déplacement du diaphragme de l'embrayage. La position du diaphragme est donc toujours fonction de la position de la pédale.

Encore un autre avantage du système de commande selon l'invention est que, comme les deux circuits amont et aval disposent d'un moyen de remise à niveau du volume de fluide, le système garde un point de fonctionnement constant quel que soit les variations de position de l'embrayage, variations qui peuvent provenir par exemple d'une usure de l'embrayage, d'un échauffement de celui-ci, ou de la commande de l'embrayage.

Selon d'autres caractéristiques de l'invention :
- le dispositif d'assistance comporte un moyen de régulation qui fait varier la valeur de la force d'assistance en fonction de la course de la pédale de commande de l'embrayage selon une loi d'assistance prédéterminée ;
- le dispositif d'assistance comporte un organe de transmission qui transmet la force d'assistance au piston d'assistance ;
- l'organe de transmission est lié en déplacement axial au piston d'assistance dans les deux sen$ de coulissement du piston ;
- l'organe de transmission coopère par contact avec une surface d'appui associée du piston d'assistance de manière que, dans le cas où la vitesse du dispositif d'assistance est inférieure à la vitesse du piston d'assistance, le dispositif d'assistance ne ralentisse pas le coulissement du piston d'assistance vers l'aval ;
- l'organe de transmission est agencé à une extrémité axiale du piston d'assistance ;
- le piston comporte un tronçon amont qui délimite la chambre amont et un tronçon aval qui délimite la chambre aval, les deux tronçons sont liés en déplacement axial par une tige de liaison, et la tige de liaison constitue l'organe de transmission du dispositif d'assista nce ;
- le circuit hydraulique étant raccordé à un réservoir de fluide en position d'embrayage, le cylindre d'assistance comporte au moins un orifice de décharge qui fait communiquer au moins une chambre hydraulique avec le réservoir de fluide, lorsque le piston d'assistance occupe sa position amont, de manière à compenser les variations de volume hydraulique dans le circuit hydraulique au cours du temps ;
- l'orifice de décharge est agencé dans le piston d'assistance et l'orifice de décharge fait communiquer la chambre amont avec la chambre aval, lorsque le piston d'assistance occupe sa position amont ;
- l'orifice de décharge comporte une soupape qui est commandée par le déplacement axial du piston d'assistance ;
- le dispositif d'assistance comporte un élément élastique qui emmagasine de l'énergie pendant la phase d'embrayage et qui restitue l'énergie au cours de la phase de débrayage pour produire la force d'assistance ;
- le moyen de régulation est un mécanisme à cames qui est entraîné par le déplacement axial du piston et qui régule la force d'assistance produite par l'élément élastique pendant la phase de débrayage ;
- le dispositif d'assistance est logé dans le corps de cylindre, et le mécanisme à cames comporte au moins une surface de commande qui est réalisée sur une paroi interne du corps de cylindre;
- l'élément élastique d'assistance est un élément élastique de compression axiale qui est interposé axialement entre une coupelle et une surface d'appui fixe par rapport au corps de cylindre d'assistance, le mécanisme à cames comporte au moins un galet mobile qui parcourt une surface de commande entre une position amont et une position aval correspondant respectivement aux positions amont et aval du piston d'assistance, et le galet mobile est lié par une première biellette au piston et par une seconde biellette à la coupelle ;
- l'axe de pivotement des biellettes sur le galet mobile est concourant à l'axe de rotation du galet ;
- la surface de commande comporte une portion amont inclinée par rapport à l'axe de coulissement, et une portion aval globalement parallèle à l'axe de coulissement de manière que, au cours d'une première partie de la phase de débrayage, le galet mobile se déplace d'abord sur la portion inclinée vers l'axe et vers l'aval, à partir de sa position amont, en transmettant une partie de l'effort de détente de l'élément élastique d'assistance au piston d'assistance, par un effet de démultiplication, puis au cours d'une deuxième partie de la phase de débrayage, le galet mobile se déplace sur la portion aval vers l'aval, suivant une direction globalement axiale, en transmettant la totalité de l'effort de détente de l'élément élastique d'assistance au piston d'assistance ;
- la distance entre les axes de pivotement de la seconde biellette est telle que, en position amont du galet mobile, le galet dépasse vers l'amont le point de la surface de commande où la seconde biellette est perpendiculaire à la surface de commande, de manière que l'effort de détente de l'élément élastique d'assistance sollicite le galet mobile vers sa position amont ;
- la dimension axiale de l'élément élastique d'assistance à l'état détendu est inférieure à la distance axiale entre la coupelle et la surface d'appui fixe associée, lorsque le piston occupe sa position aval, de manière à suspendre la force d'assistance pendant la fin de la course du piston vers l'aval ;
- le dispositif d'assistance comporte un actionneur électrique qui commande la détente de l'élément élastique pendant la phase de débrayage ;
- le moyen de régulation du dispositif d'assistance est une unité électronique de pilotage qui commande l'actionneur électrique ;
- l'élément élastique d'assistance est un ressort hélicoïdal de compression ;
- le dispositif d'assistance est raccordé à une source d'énergie qui est externe au système de commande et qui est embarquée dans le véhicule que le système de commande équipe, et ladite énergie produit la force d'assistance qui est transmise au piston ;
- le dispositif d'assistance comporte un actionneur électrique qui est commandé de manière à transmettre une force d'assistance au piston pendant la phase de débrayage.
- le moyen de régulation du dispositif d'assistance est une unité électronique de pilotage qui commande l'actionneur électrique produisant la force d'assistance ;
- le dispositif d'assistance comporte un vérin qui est raccordé à une source de pression hydraulique ou pneumatique et qui transmet une force d'assistance au piston pendant la phase de débrayage ;
- le moyen de régulation du dispositif d'assistance comporte au moins un distributeur qui est interposé entre le vérin et la source de pression hydraulique ou pneumatique ;
- le moyen de régulation comporte un distributeur à deux positions qui est raccordé à une source de pression pour former une vanne de charge et un distributeur à deux positions qui est raccordé à un réservoir de fluide pour former une vanne de décharge, et chaque distributeur est commandé par la pression hydraulique dans le circuit amont, de manière que la pression hydraulique dans le circuit amont tende vers une première valeur constante lors d'une course de débrayage, et tende vers une seconde valeur constante, inférieure à la première valeur, lors d'une course d'embrayage ;
- le moyen de régulation comporte un distributeur à trois positions, une position de charge qui est raccordée à une source de pression, une position intermédiaire de fermeture, une position de décharge qui est raccordée à un réservoir de fluide, et le distributeur est commandé, du côté de la position de charge, par la pression hydraulique dans le circuit amont, et du côté de la position de décharge par la pression hydraulique dans le circuit aval, de manière que la force d'assistance appliquée sur le piston d'assistance pendant la phase de débrayage soit proportionnelle à la pression hydraulique dans le circuit aval ;
- le distributeur est commandé par une unité électronique de pilotage ;
- le piston comporte au moins un élément élastique qui rappelle le piston vers sa position amont.

D'autres caractéristiques et avantages de l'invention apparaîtront à la lecture de la description détaillée qui suit pour la compréhension de laquelle on se reportera aux dessins annexés dans lesquels :
- la figure 1 est un schéma qui représente un système de commande hydraulique d'embrayage réalisé conformément aux enseignements de l'invention ;
- les figures 2 et 3 illustrent schématiquement le principe de fonctionnement de l'assistance dans le système de commande selon l'invention ;
- la figure 4 est un schéma similaire à celui de la figure 2 qui illustre une variante de réalisation de la mise au réservoir du circuit hydraulique de commande ;
- la figure 5 est une vue en coupe axiale qui représente schématiquement le cylindre d'assistance du système de commande selon un premier mode de réalisation de l'invention dans lequel le dispositif d'assistance comporte un mécanisme à cames, le piston étant représenté respectivement dans sa position amont et dans sa position aval ;
- les figures 6 et 7 sont des vues partielles en coupe axiale qui représentent un détail de la figure 5 et qui illustrent deux positions intermédiaires successives d'un galet mobile équipant le mécanisme à cames ;
- la figure 8 est un diagramme illustrant l'évolution de la pression hydraulique dans le circuit de commande et l'évolution de la force d'assistance en fonction de la course de la pédale d'embrayage ;
- la figure 9 est une vue similaire à celle de la figure 5 qui représente schématiquement un cylindre d'assistance selon un deuxième mode de réalisation dans lequel le dispositif d'assistance est agencé à l'extrémité amont du cylindre d'assistance ;
- la figure 10 est un schéma similaire à celui de la figure 2 qui représente schématiquement un système de commande selon un troisième mode de réalisation de l'invention dans lequel la détente du ressort d'assistance est commandée par un actionneur électrique ;
- la figure 11 est un schéma similaire à celui de la figure 9 qui représente un cylindre d'assistance selon un quatrième mode de réalisation du système de commande selon l'invention dans lequel la force d'assistance est produite par un moteur électrique ;
- la figure 12 est un schéma similaire à celui de la figure 2 qui représente schématiquement un système de commande selon un cinquième mode de réalisation de l'invention dans lequel la force d'assistance est produite par un vérin raccordé à une source de pression ;
- la figure 13 est une vue en coupe axiale qui représente un cylindre d'assistance adapté au système de commande de la figure 12 et comportant un vérin à membrane ;
- les figures 14, 16, et 18 sont des schémas similaires à celui de la figure 2 qui illustrent trois solutions différentes de régulation de la force d'assistance adaptées au système de commande de la figure 12 ;
- les figures 15 et 17 sont des diagrammes qui illustrent les lois d'assistance associées respectivement aux deux premières solutions représentées sur les figures 14 et 16 ;
- la figure 19 est un vue en coupe axiale qui représente une variante du mode de réalisation de la figure 13.

Dans la description qui va suivre, des éléments identiques, similaires ou analogues seront désignés par les mêmes chiffres de référence.

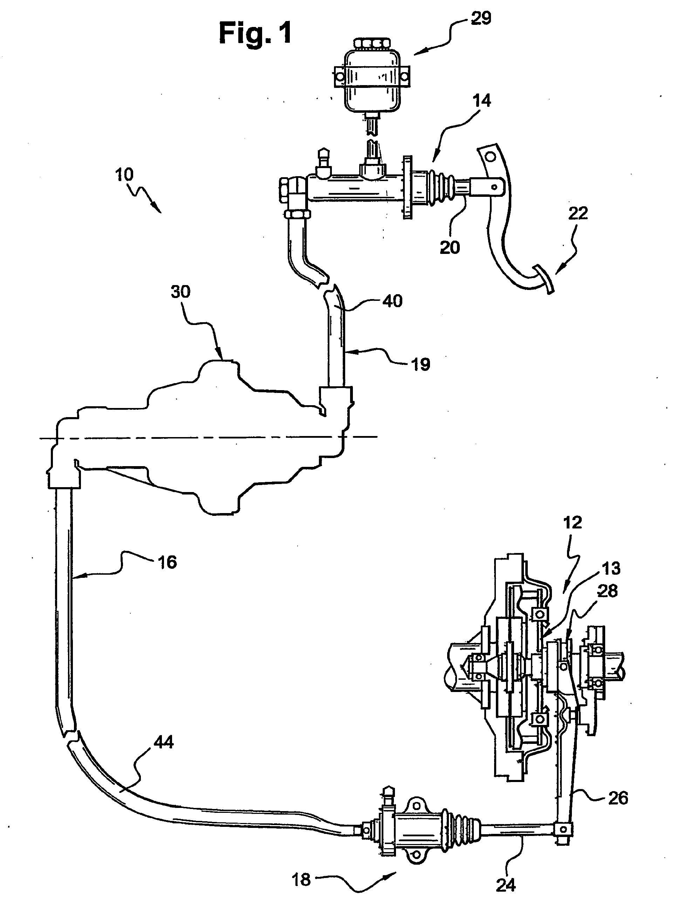

Sur la figure 1, on a représenté un système de commande hydraulique 10 d'un embrayage 12 de véhicule automobile réalisé conformément aux enseignements de l'invention.

Le système de commande 10 comporte un cylindre émetteur amont 14 relié par une canalisation ou conduite 16 à un cylindre récepteur aval 18 de structure similaire au cylindre émetteur 14.

Le cylindre émetteur 14, le cylindre récepteur 18, et la conduite 16 forment un circuit hydraulique de commande 19.

Chaque cylindre émetteur 14 ou récepteur 18 comporte un piston (non représenté) mobile axialement à l'intérieur d'un corps de cylindre pour délimiter une chambre hydraulique de volume variable. Un orifice de raccordement, sur lequel se branche la conduite 16, débouche dans la chambre hydraulique.

Le cylindre émetteur 14 comporte une tige de piston 20 qui est reliée ici à une pédale d'embrayage 22 sur laquelle agit le conducteur du véhicule.

Le piston du cylindre émetteur 14 est prévu pour expulser un fluide ou liquide de commande contenu dans la chambre hydraulique en direction de la conduite 16, lors d'une opération de débrayage.

Lorsque l'embrayage 12 est engagé, le volume de la chambre hydraulique du cylindre émetteur 14 est maximum tandis que le volume de la chambre hydraulique du cylindre récepteur 18 est minimum.

Lors de l'opération de débrayage, le volume de la chambre hydraulique du cylindre émetteur 14 diminue, tandis que le volume de la chambre hydraulique du cylindre récepteur 18 augmente.

Le piston du cylindre récepteur 18 provoque alors le déplacement d'une tige 24, qui agit ici sur une fourchette de débrayage 26 actionnant une butée de débrayage 28.

Lorsque le conducteur relâche son action sur la pédale d'embrayage 22, le piston du cylindre récepteur 18 est ramené vers sa position initiale par un ressort d'embrayage tel qu'un diaphragme 13.

En revenant vers sa position initiale, le cylindre récepteur 18 repousse la colonne d'huile contenue dans le circuit hydraulique 19, ce qui provoque le rappel du piston du cylindre émetteur 14 vers sa position initiale.

La pédale d'embrayage 22 est rappelée vers sa position initiale par un ressort de rappel et/ou par le retour du piston du cylindre émetteur 14.

Généralement, chaque cylindre émetteur 14 et récepteur 18 comporte un ressort (non représenté) qui agit entre le piston et le fond du corps du cylindre, et qui garantit le retour du piston jusqu'à sa position initiale en butée.

De préférence, la chambre hydraulique du cylindre émetteur 14 est susceptible d'être raccordée à un réservoir de fluide 29, de manière à compenser les variations de volume du circuit hydraulique 19 au cours du temps.

A cet effet, la chambre hydraulique du cylindre émetteur 14 comporte au moins un orifice de décharge (non représenté) qui est ouvert lors du retour complet du piston émetteur dans sa position initiale, et qui fait communiquer le circuit hydraulique 19 avec le réservoir 29.

On note que, pendant la phase de débrayage, la pédale 22 comporte une course morte au début de son pivotement, qui correspond à un déplacement du piston émetteur jusqu'à la position axiale dans laquelle il ferme l'orifice de décharge.

Pendant la course morte, le piston émetteur repousse le fluide vers le réservoir 29, sans provoquer de déplacement du piston récepteur.

Conformément aux enseignements de l'invention, le système de commande 10 comporte un cylindre d'assistance 30 qui est interposé dans la conduite 16, entre le cylindre émetteur 14 et le cylindre récepteur 18.

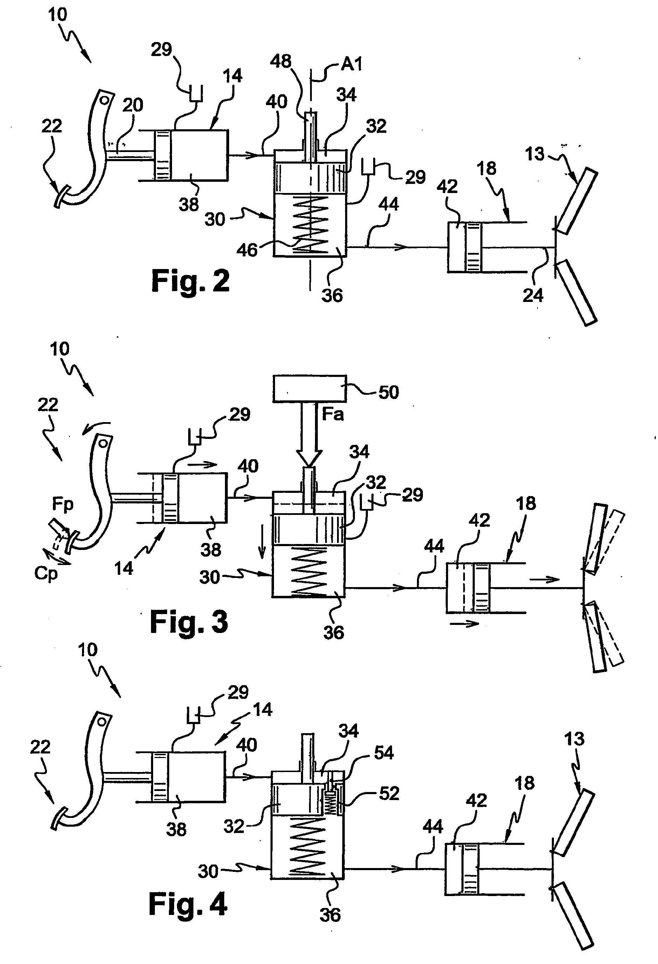

Sur la figure 2, on a représenté le système de commande 10 selon l'invention de manière simplifiée.

On note que, sur cette figure, la tige 24 du piston récepteur agit directement sur le diaphragme 13 de l'embrayage 12 par l'intermédiaire d'une butée comportant un roulement à billes (non représenté).

Le cylindre d'assistance 30 comporte un piston d'assistance 32 qui est monté coulissant suivant un axe principal A1, entre une position amont et une position aval, de manière à délimiter une chambre hydraulique amont 34 et une chambre hydraulique aval 36 de volumes variables en fonction de la position axiale du piston 32.

La chambre amont 34 communique avec la chambre 38 du cylindre émetteur 14 par une portion amont 40 du circuit hydraulique 19, et la chambre aval 36 communique avec la chambre 42 du cylindre récepteur 18 par une portion aval 44 du circuit hydraulique 19.

Le circuit hydraulique amont 40 est raccordé au réservoir de fluide 29 au niveau de la chambre émetteur 38.

Le circuit hydraulique aval 44 est raccordé à un réservoir de fluide, par exemple le même réservoir 29 que le circuit hydraulique amont 40, ici au niveau de la chambre aval 36 du cylindre d'assistance 30.

Selon le mode de réalisation illustré ici, le cylindre d'assistance 30 comporte un ressort 46 qui est interposé axialement entre le piston d'assistance 32 et le fond de la chambre aval 36, et qui rappelle le piston d'assistance 32 vers sa position amont.

Sur le schéma, le piston d'assistance 32 comporte une tige 48 qui s'étend vers l'extérieur à travers la chambre amont 34.

Selon une variante (non représentée), le cylindre d'assistance 30 avec son piston 32 peut être remplacé par une chambre comportant une membrane intermédiaire séparant les chambres amont 34 et aval 36, et jouant le rôle du piston d'assistance 32. Cette membrane comporte de la même manière la tige 48.

Conformément aux enseignements de l'invention, pendant la phase de débrayage, qui est illustrée par la figure 3, une force d'assistance Fₐ est appliquée sur le piston d'assistance 32, ici par l'intermédiaire de la tige 48, de manière à soulager l'effort d'appui Fp de l'utilisateur sur la pédale 22.

La force d'assistance Fₐ est produite par un dispositif d'assistance 50 qui sera décrit par la suite.

Selon une variante de réalisation représentée sur la figure 4, un orifice de décharge 52 est réalisé dans le piston d'assistance 32, de manière à faire communiquer le circuit hydraulique amont 40 avec le circuit hydraulique aval 44, lorsque le piston 32 occupe sa position amont.

Selon le schéma de la figure 4, l'orifice de décharge 52 traverse axialement le piston 32 et il comporte une soupape de décharge 54 qui est sollicitée élastiquement vers sa position de fermeture, et qui s'ouvre mécaniquement lorsque le piston d'assistance 32 vient occuper sa position amont, par appui d'une tige de la soupape 54 sur le fond de la chambre amont 34.

L'agencement de l'orifice de décharge 52 dans le piston 32 permet notamment de raccorder tout le circuit hydraulique 19 au réservoir de fluide 29, avec un seul raccordement, agencé ici au niveau du cylindre émetteur 14.

Cet agencement permet aussi de ne pas ajouter une course morte supplémentaire à la pédale d'embrayage 22, ce qui est le cas lorsque le cylindre d'assistance 30 est raccordé au réservoir 29 comme sur la figure 2.

L'ouverture de la soupape de décharge 54 peut être calibrée de manière qu'une augmentation brutale de la pression hydraulique dans la chambre émetteur 38 provoque, presque aussitôt, un déplacement du piston d'assistance 32 vers l'aval, le fluide n'ayant pas le temps de s'écouler par la soupape de décharge 54, et donc la fermeture de l'orifice de décharge 52. Ce calibrage peut permettre de choisir la valeur de la première course axiale du piston d'assistance 32 vers l'aval, avant que le dispositif d'assistance 50 ait commencé à appliquer une force d'assistance Fₐ.

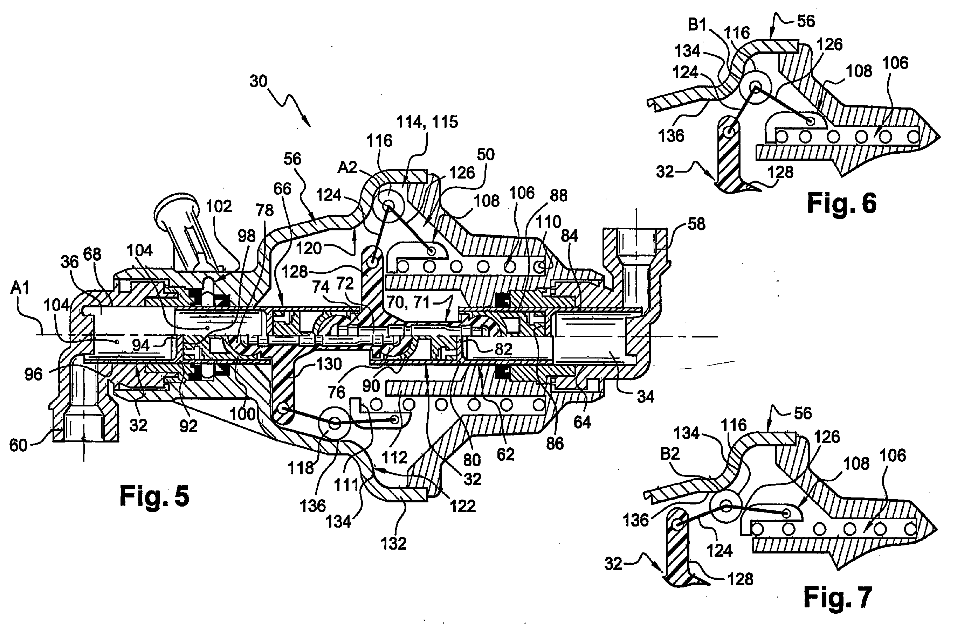

On décrit maintenant un premier mode de réalisation du système de commande 10 selon l'invention, qui est représenté sur les figures 5 à 7, dans lequel le dispositif d'assistance 50 comporte un élément élastique qui emmagasine de l'énergie pendant la phase d'embrayage et qui restitue l'énergie, sous la forme d'une force d'assistance Fₐ, au cours de la phase de débrayage.

Selon le premier mode de réalisation, le cylindre d'assistance 30 comporte un corps de cylindre 56 qui est doté d'un orifice d'entrée 58 et d'un orifice de sortie 60.

Le piston d'assistance 32 est monté coulissant, suivant l'axe principal A1, à l'intérieur du corps de cylindre 56 qui a globalement une forme tubulaire d'axe A1.

Dans la suite de la description, des éléments seront qualifiés d'interne ou d'externe par rapport à l'axe principal A1 suivant une direction radiale.

En considérant la figure 5, le piston 32 est représenté en position amont sur la demi-coupe supérieure, et en position aval sur la demi-coupe inférieure.

La chambre amont 34 communique avec le cylindre émetteur 14 à travers l'orifice d'entrée 58, et la chambre aval 36 communique avec le cylindre récepteur 18 à travers l'orifice de sortie 60.

Selon le mode de réalisation représenté ici, le piston d'assistance 32 est réalisé en plusieurs parties.

Le piston 32 comporte un tronçon amont 62, qui est prévu pour coulisser axialement dans un alésage amont complémentaire 64, et un tronçon aval 66, qui est prévu pour coulisser axialement dans un alésage aval complémentaire 68, les deux tronçons 62, 86 étant liés en déplacement axial par une tige axiale de liaison 70.

La tige de liaison 70 comporte ici une tige interne 72, par exemple en métal, et un corps externe 74 moulé sur la tige interne 72.

L'extrémité amont 76 et l'extrémité aval 78 de la tige 70 ont ici chacune la forme d'une tête sphérique.

Les deux tronçons 62, 66 sont ici de formes globalement identiques.

Le tronçon amont 62 a globalement une forme tubulaire à profil axial en "H", c'est-à-dire qu'il comporte deux parties tubulaires sensiblement symétriques par rapport à une paroi transversale de séparation 80.

Du côté de la face amont 82 de la paroi transversale 80, le tronçon amont 62 forme une chemise 84 qui délimite une partie de la chambre amont 34.

Du côté de la face aval 86 de la paroi transversale 80, le tronçon amont 62 forme un logement 88 qui reçoit une pièce globalement cylindrique 90 formant réceptacle pour la liaison entre l'extrémité axiale amont 76 de la tige de liaison 70 et le tronçon amont 62 du piston 32.

Selon le mode de réalisation représenté ici, le tronçon aval 66 est sensiblement similaire au tronçon amont 62, et le tronçon aval 66 est agencé de manière sensiblement symétrique au tronçon amont 62, par rapport à un plan transversal de symétrie.

Ainsi, le tronçon aval 66 comporte une paroi transversale de séparation 92, et forme, du côté de la face aval 94 de cette paroi 92, une chemise 96 qui délimite une partie de la chambre aval 3fi.

Du côté de la face amont 98 de la paroi transversale 92, le tronçon aval 66 reçoit une pièce cylindrique 100, similaire à celle du tronçon amont 62, formant réceptacle pour la liaison entre l'extrémité axiale aval 78 de la tige de liaison 70 et le tronçon aval 66.

Dans le mode de réalisation représenté, l'alésage aval 68 comporte une gorge radiale annulaire 102 qui communique avec le réservoir de liquide 29, de manière à former un orifice de décharge. Ce mode de réalisation correspond donc au mode de réalisation représenté sur les figures 2 et 3, dans lequel le circuit aval 44 comporte une mise au réservoir 29 au niveau de la chambre aval 36.

La chemise 96 du tronçon aval 66 comporte ici plusieurs orifices radiaux 104 qui sont sensiblement alignés circonférentiellement et qui sont agencés en vis-à-vis de la gorge radiale 102, lorsque le piston 32 occupe sa position amont, telle que représentée dans la moitié supérieure de la figure 5.

Les orifices radiaux 104 permettent de mettre en communication la chambre aval 36 et le réservoir 29, lorsque le piston 32 occupe sa position amont, de manière à compenser les, variations de volume hydraulique dans le circuit aval 44 au cours du temps.

Dans la position aval du piston 32, telle que représentée dans la moitié inférieure de la figure 5, les orifices 104 sont décalés axialement vers l'aval, par rapport à la gorge radiale 102, de sorte que la chambre aval 36 ne communique pas avec le réservoir 29.

Bien entendu, comme indiqué en référence avec la variante de la figure 4, la gorge radiale 102 et les orifices radiaux 104 peuvent être supprimés au profit d'un orifice de décharge 52 réalisé axialement dans le piston 32 et muni d'une soupape de décharge 54.

Le dispositif d'assistance 50 comporte ici un élément élastique sous la forme d'un ressort hélicoïdal de compression axiale 106.

Le ressort 106 est prévu pour se comprimer pendant la phase d'embrayage, sous l'effet du retour du piston d'assistance 32 dans sa position amont, de manière à emmagasiner de l'énergie, et il est prévu pour restituer cette énergie au cours de la phase de débrayage en produisant une force d'assistance Fₐ.

Le ressort 106 est interposé axialement entre une coupelle annulaire 108 mobile axialement et une surface radiale annulaire d'appui fixe 110 aménagée dans le corps de cylindre 56.

La coupelle 108 comporte une surface radiale annulaire d'appui 111 qui est orientée vers l'amont et qui fait face à la surface d'appui fixe 110, orientée vers l'aval.

Le ressort 106 est ici monté autour d'une portion tubulaire interne de guidage 112 du corps de cylindre 56.

Le dispositif d'assistance 50 comporte un mécanisme à cames 114 qui est entraîné par le déplacement axial du piston 32, et qui forme un moyen de régulation 115 permettant de faire varier la valeur de la force d'assistance Fₐ en fonction de la course Cp de la pédale 22 selon une loi d'assistance prédéterminée.

Le mécanisme à cames 114 comporte, par exemple, deux galets mobiles 116, 118 qui parcourent chacun une surface de commande associée 120, 122.

Les deux galets 116, 118 sont ici agencés de part et d'autre du piston 32, et ils sont diamétralement opposés.

L'axe de rotation A2 de chaque galet 116, 118 est sensiblement orthogonal à l'axe de coulissement A1 du piston 32.

Chaque galet 116, 118 est lié par une biellette aval 124 à la tige de liaison 70 et par une biellette amont 126 à la coupelle 108.

On note que la tige de liaison 70 constitue ici un organe de transmission 71 qui permet au dispositif d'assistance 50 de transmettre la force d'assistance Fₐ au piston d'assistance 32.

Du côté du galet 116, 118, les biellettes 124, 126 sont montées à pivotement autour de l'axe de rotation A2 du galet 116, 118.

La biellette aval 124 est montée à pivotement sur l'extrémité libre d'un bras transversal associé 128, 130 de la tige de liaison 70.

Selon le mode de réalisation représenté ici, le corps de cylindre 56 forme une enveloppe 132 globalement cylindrique autour du dispositif d'assistance 50. L'enveloppe 132 est étagée en diamètre.

Avantageusement, la surface de commande 120, 122 associée à chaque galet 116, 118 est réalisée sur la paroi interne de l'enveloppe 132.

Les surfaces de commande 120, 122 sont ici sensiblement symétriques par rapport à un plan axial (A1) et elles s'étendent globalement dans un même plan axial.

Chaque surface de commande 120, 122 comporte une portion amont 134 inclinée par rapport à l'axe de coulissement A1, et une portion aval 136 globalement parallèle à l'axe de coulissement A1.

La portion amont 134 a ici un profil arrondi convexe vers l'axe A1 et vers l'amont.

Selon une forme de réalisation avantageuse, la distance entre les axes de pivotement de chaque biellette amont 126, 128 est telle que, en position amont du galet mobile 116, 118 associé, le galet dépasse vers l'amont le point B1 de la surface de commande . 120, 122 où la biellette amont 126, 128 est perpendiculaire à la surface de commande 120, 122, de manière que l'effort de détente du ressort d'assistance 106 sollicite le galet mobile 116, 118 vers sa position amont.

Selon une autre forme de réalisation avantageuse, lorsque le piston 32 occupe sa position aval de débrayage, la distance axiale entre la surface d'appui 111 de la coupelle 108 et la surface d'appui fixe 110 est supérieure à la dimension axiale du ressort 106 à l'état détendu, de sorte que le ressort 106 ne sollicite pas axialement le piston 32 vers sa position aval.

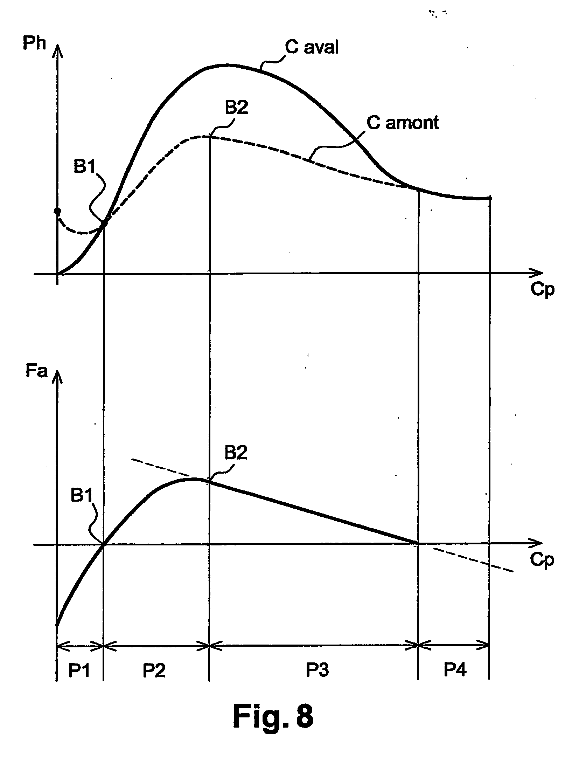

On explique maintenant le fonctionnement du mécanisme à cames 114 selon l'invention, en considérant notamment les positions partielles représentées sur les figures 6 et 7, et les diagrammes représentés sur la figure 8.

Sur la partie haute de la figure 8, la courbe Cₐᵥₐₗ en trait continu représente l'évolution de la pression hydraulique Pₕ dans la chambre aval du cylindre d'assistance 30, pendant la phase de débrayage, en fonction de la course Cp de la pédale d'embrayage 22, et la courbe Cₐₘₒₙₜ en trait discontinu représente l'évolution de la pression hydraulique Pₕ dans la chambre amont du cylindre d'assistance 30, pendant la phase de débrayage, en fonction de la course Cp de la pédale d'embrayage 22.

Sur la partie basse de la figure 8, la courbe en trait continu représente l'évolution de la force d'assistance Fₐ produite par le ressort d'assistance 106, pendant la phase de débrayage, en fonction de la course Cp de la pédale d'embrayage 22, et la droite en trait discontinu représente la raideur du ressort d'assistance 106.

Dans la position amont du piston d'assistance 32, qui est illustrée par la partie haute de la figure 5, le ressort 106 est comprimé et il sollicite axialement (A1) chaque biellette amont 126, ainsi que le galet associé 116, 118, vers la surface de commande 120, 122 et vers l'extérieur, sans provoquer de déplacement du piston d'assistance 32.

Les galets mobiles 116, 118 sont ici retenus par la biellette aval 124 associée qui est liée au piston d'assistance 32 en position amont de butée.

Au début de la phase de débrayage, l'utilisateur appuie sur la pédale 22 de commande de l'embrayage 12 de manière à déplacer le piston du cylindre émetteur 14 vers l'aval.

La première partie du déplacement du piston du cylindre émetteur 14 correspond à une course morte, jusqu'à la fermeture de l'orifice de décharge raccordant la chambre émetteur 38 au réservoir 29.

En poursuivant son déplacement vers l'aval, le piston du cylindre émetteur 14 provoque ensuite une augmentation de la pression hydraulique Pₕ dans la chambre amont 34 du cylindre d'assistance 30, ce qui entraîne un déplacement axial A1 du piston d'assistance 32 vers l'aval.

Le déplacement du piston d'assistance 32 entraîne un déplacement des galets 116, 118 sur les surfaces de commande associées 120, 122, vers l'intérieur et vers l'aval.

Au cours d'une première phase P1 de son déplacement axial, le piston d'assistance 32 provoque une compression supplémentaire du ressort d'assistance 106, de sorte que le dispositif d'assistance 50 produit un effort de résistance qui s'oppose au déplacement de la pédale d'embrayage 22, ce qui correspond à une force d'assistance Fₐ négative, illustrée par la partie inférieure de la figure 8.

Au cours de cette première phase P1, la pression hydraulique Pₕ dans la chambre amont 34 du cylindre d'assistance 30 est supérieure à la pression hydraulique Pₕ dans la chambre aval 36.

La première phase P1 du déplacement du piston 32 se termine lorsque les galets 116, 118 atteignent le point B1 de la surface de commande 120, 122 où la biellette amont 126, 128 est perpendiculaire à la surface de commande 120, 122, ce qui est représenté sur la figure 6.

Au moment où les galets 116, 118 atteignent ce point B1 de la surface de commande 120, 122, la force de détente du ressort d'assistance 106 est annulée par la force de réaction de la surface de commande 120, 122, de sorte que la pression hydraulique Pₕ s'égalise entre la chambre amont 34 et la chambre aval 36 du cylindre d'assistance 30.

On note que, pendant la première phase P1, le déplacement axial du piston 32 provoque la fermeture du raccordement 102 de la chambre aval 36 avec le réservoir 29, grâce au décalage axial des orifices radiaux 104 vers l'aval.

Pendant une deuxième phase P2 du déplacement axial du piston d'assistance 32 vers l'aval, le ressort d'assistance 106 commence à se détendre en produisant une force d'assistance Fₐ sur le piston d'assistance 32.

La force d'assistance Fₐ produite par le ressort 106 est démultipliée par le mécanisme à cames 114, en fonction du profit de la portion amont 134 de la surface de commande 120, 122, ce qui permet de réguler la force d'assistance Fₐ en fonction de la -course Cp de la pédale 22, selon une loi d'assistance prédéterminée.

La deuxième phase P2 du déplacement du piston 32 se termine lorsque les galets 116, 118 atteignent l'extrémité aval B2 de la portion amont 134 de la surface de commande associée 120, 122, tel que représenté sur la figure 7.

Le piston d'assistance 32 entame alors une troisième phase P3 de son déplacement axial au cours de laquelle les galets 116, 118 parcourent la portion aval 136 de la surface de commande associée 120, 122.

Pendant cette troisième phase P3, le ressort d'assistance 106 transmet la totalité de sa force de détente au piston d'assistance 32, puisque les biellette 124, 126 ne pivotent plus et que les galets 116, 118 ne sont plus retenus axialement par la surface de commande 120, 122.

On note que, pendant la troisième phase P3, les biellettes 124, 126 peuvent être proches d'une position alignée mais il est préférable de conserver un angle minimum d'inclinaison entre les biellettes 124, 126, comme sur la figure 7 et sur la partie inférieure de la figure 5, de manière à inciter les biellettes 124, 126 à pivoter, pendant le retour du piston d'assistance 32 vers sa position amont, pour éviter un blocage du piston 32 dans le cylindre 30.

Selon la forme de réalisation avantageuse prévue ici, comme la distance axiale entre la surface d'appui 111 de la coupelle 108 et la surface d'appui fixe 110 est supérieure à la dimension axiale du ressort 106 à l'état détendu, la troisième phase P3 est suivie d'une quatrième phase P4 au cours de laquelle le piston d'assistance 32 continue de coulisser jusqu'à sa position de butée aval, sans bénéficier d'une force d'assistance Fₐ puisque le ressort d'assistance 106 est à l'état détendu.

La quatrième phase P4 est utile pour minimiser les forces de frottement induites par le dispositif d'assistance 50 pendant le déplacement du piston d'assistance 32, de manière à garantir le retour du piston 32 vers l'amont, à partir de sa position aval, notamment lorsque la force de rappel du piston d'assistance 32 produite, par exemple, par le diaphragme 13 est faible.

Lorsque l'utilisateur relâche son appui sur la pédale 22, les éléments de rappel de l'embrayage 12 tels que le diaphragme 13, provoquent le retour du piston d'assistance 32 vers l'amont.

Le retour du piston 32 vers l'amont provoque un retour du mécanisme à cames 114 dans sa position initiale et une compression du ressort d'assistance 106, ce qui lui permet d'emmagasiner de l'énergie élastique.

De préférence, à la fin de la course du piston d'assistance 32 vers l'amont, ce qui correspond à la première phase P1 du déplacement du piston 32 vers l'aval, le ressort d'assistance 106 provoque un rappel élastique du piston 32 jusqu'à sa position amont, en sollicitant les galets mobiles 116, 118 vers leurs positions amont de repos.

En position amont du piston 32, les orifices 104 de son tronçon aval 66 viennent se positionner en vis-à-vis de la gorge radiale 102, ce qui permet une mise au réservoir de liquide 29 du circuit hydraulique aval 44.

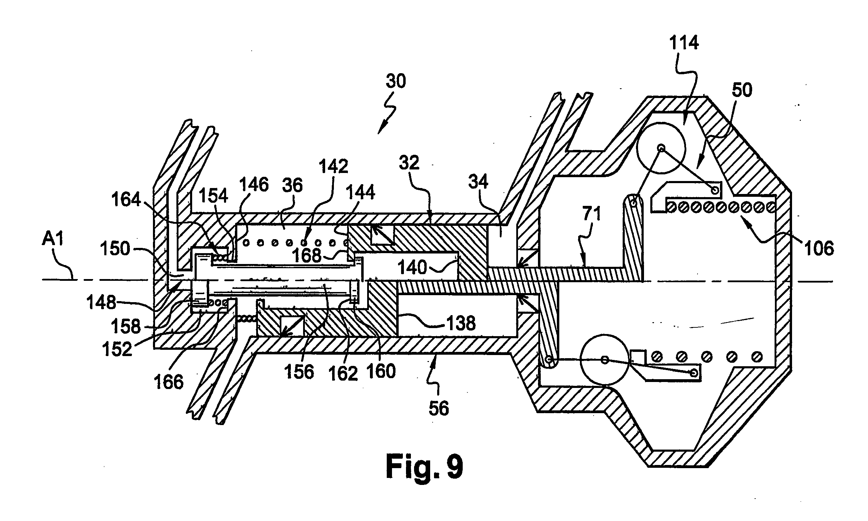

Sur la figure 9, on a représenté schématiquement un deuxième mode de réalisation du système de commande 10 selon l'invention.

On note que la représentation du deuxième mode de réalisation a été simplifiée par rapport à la représentation du premier mode de réalisation sur la figure 5.

Ce deuxième mode de réalisation se différencie du premier principalement par le fait que le dispositif d'assistance 50 est agencé à l'extrémité axiale amont du cylindre d'assistance 30, et non pas entre les deux chambres hydrauliques 34, 36.

Selon ce mode de réalisation, le cylindre d'assistance 30 comporte un piston 32 dont la face transversale amont 138 délimite la chambre amont 34 et dont la face transversale aval 140 délimite la chambre aval 36.

Le piston 32 est ici réalisé globalement en une seule pièce.

Selon le mode de réalisation représenté ici, un ressort hélicoïdal de compression 142 est interposé axialement entre une surface transversale d'extrémité aval 144 du piston 32 et la paroi de fond 146 de la chambre aval 36. Ce ressort 142 sert à garantir le rappel du piston 32 jusqu'à sa position amont de butée.

Le dispositif d'assistance 50 est réalisé de manière similaire à celui du premier mode de réalisation. Il comporte notamment un ressort d'assistance 106 et un mécanisme à cames 114.

Le dispositif d'assistance 50 comporte un organe de transmission 71 sous la forme d'une tige de transmission qui s'étend axialement vers la face transversale amont 138 du piston d'assistance 32.

Selon une forme de réalisation avantageuse, qui est représentée ici, la tige de transmission 71 coopère uniquement par contact avec la surface transversale amont 138 du piston d'assistance 32.

Cette configuration de la tige de transmission 71 permet au piston d'assistance 32 de coulisser indépendamment de la tige 71. Ainsi, dans le cas où la pression exercée par le dispositif d'assistance 50 sur la tige de transmission 71 est inférieure à la pression exercée sur le piston d'assistance 32 par le fluide contenu dans la chambre amont 34, le piston d'assistance 32 peut coulisser vers l'aval sans être ralenti par le mouvement du dispositif d'assistance 50.

Une telle configuration permet aussi de palier à un dysfonctionnement du dispositif d'assistance 50, car le système de commande 10 peut fonctionner sans assistance.

On a aussi représenté, sur la figure 9, une variante de réalisation du dispositif de mise au réservoir 29 de la chambre aval 36.

Avantageusement, le cylindre , d'assistance 30 comporte ici une soupape de décharge 148 qui est commandée par le piston 32, de manière à effectuer la mise au réservoir 29 lorsque le piston 32 occupe sa position amont.

A cet effet, le conduit de raccordement au réservoir 29 débouche, par un orifice de décharge 150, dans une cavité cylindrique intermédiaire 152 qui est agencée à l'extrémité axiale aval du corps de cylindre 56.

La cavité intermédiaire 152 communique avec la chambre aval 36 par une ouverture 154 qui débouche dans la paroi de fond 146 de la chambre aval 36.

La soupape 148 comporte une tige ou queue 156 qui est munie, à son extrémité axiale aval, d'une tête 158 susceptible d'obturer l'orifice de communication 150, et à son extrémité axiale amont, d'une collerette de commande 160 délimitant une surface transversale d'appui 162 orientée vers l'aval.

La soupape 148 est sollicitée axialement vers l'aval, donc vers la position d'obturation de l'orifice de décharge 150, par un ressort de soupape 164 qui est interposé axialement entre la tête 158 et un rebord annulaire transversal 166, orienté vers l'aval, de la cavité intermédiaire 152.

La collerette de commande 160 de la soupape 148 est prévue pour coopérer par contact avec la surface transversale amont d'un rebord annulaire transversal 168 agencé à l'extrémité axiale aval du piston 32 de manière que, en fin de course du piston 32 vers l'amont, le rebord annulaire 168 vienne en appui axial contre la surface transversale 182 de la collerette de commande 160 pour provoquer un déplacement axial de la soupape 148 vers l'amont, à l'encontre de son ressort 164.

Le déplacement de la soupape 148 vers l'amont provoque l'ouverture de l'orifice de décharge 150 ce qui permet la mise au réservoir 29 de la chambre aval 36, à la fin de la course du piston 32 vers l'amont.

Le fonctionnement du cylindre d'assistance 30 selon le deuxième mode de réalisation est similaire à celui du cylindre d'assistance 30 selon le premier mode de réalisation.

Par rapport au premier mode de réalisation, la soupape de décharge 148 selon le deuxième mode de réalisation a l'avantage de nécessiter une course axiale plus faible pour provoquer la mise au réservoir 29 de la chambre aval 36, avec une section de passage du fluide suffisante.

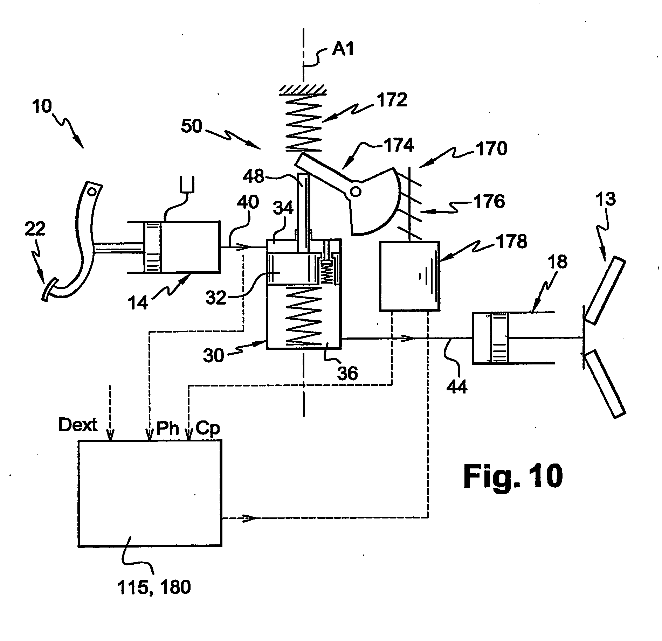

Sur la figure 10, on a représenté schématiquement un troisième mode de réalisation du système de commande 10 selon l'invention, dans lequel le dispositif d'assistance 50 comporte un actionneur électrique 170 qui commande la détente d'un élément élastique d'assistance 172 pendant la phase de débrayage.

Selon ce mode de réalisation, la force d'assistance Fₐ est donc produite par la détente d'un élément élastique d'assistance 172, ici un ressort hélicoïdal de compression, comme dans le premier et le deuxième mode de réalisation.

Le mécanisme à cames 114 a ici été remplacé par l'actionneur électrique 170.

Dans le schéma de la figure 10, l'actionneur électrique 170 comporte un levier 174 qui est interposé axialement entre la tige 48 du piston d'assistance 32 et l'extrémité axiale mobile du ressort d'assistance 172, et qui est commandé en pivotement par l'arbre de transmission 176 d'un moteur électrique 178.

Le ressort d'assistance 172 emmagasine de l'énergie élastique pendant la phase d'embrayage, notamment sous l'effet du retour élastique de l'embrayage 12 vers sa position d'embrayage qui repousse le piston d'assistance 32 vers sa position amont.

Pendant la phase de débrayage, l'actionneur électrique 170 libère le ressort d'assistance 172 de manière à produire la force d'assistance Fₐ sur le piston- 32.

De préférence, le moteur 178 est commandé par une unité électronique de pilotage 180 qui constitue un moyen de régulation 115 de la force d'assistance Fₐ.

L'unité de pilotage 180 commande, par exemple, le moteur électrique 178, en fonction de paramètres de fonctionnement tels que :
- la pression hydraulique Pₕ dans le circuit amont 40,
- la course Cp de la pédale d'embrayage 22,
- des données externes Dₑₓₜ au système de commande 10, par exemple des données relatives au fonctionnement du moteur du véhicule, au fonctionnement de la boîte de vitesse du véhicule, au fonctionnement de l'embrayage 12, etc.

Les paramètres de fonctionnement peuvent être fournis à l'unité de pilotage 180 par des capteurs (non représentés).

La valeur de la course Cp de la pédale 22 peut être fournie à l'unité de pilotage 180 par le moteur électrique 178, notamment dans le cas où la rotation de son arbre de transmission est liée au coulissement du piston d'assistance 32.

L'unité de pilotage 180 peut moduler la force d'assistance Fₐ selon au moins une loi d'assistance prédéterminée.

On décrit maintenant un quatrième et un cinquième modes de réalisation du système de commande 10 selon l'invention dans lesquels le dispositif d'assistance 50 est raccordé à une source d'énergie 182, 184 qui est extérieure au système de commande 10 et qui est embarquée dans le véhicule que le système de commande 10 équipe.

Selon ces modes de réalisation, la force d'assistance Fₐ est produite par la source d'énergie extérieure 182, 184 puis elle est transmise au piston d'assistance 32.

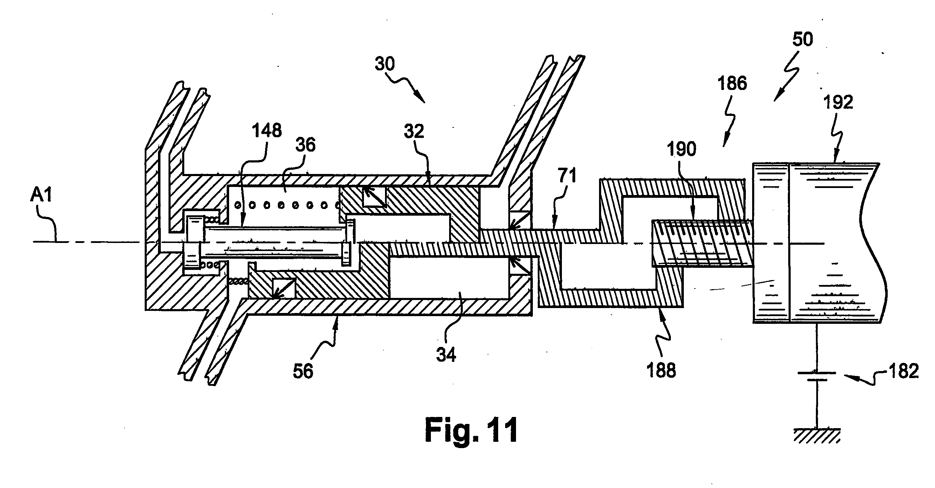

Dans le quatrième mode de réalisation, qui est représenté sur la figure 11, la source d'énergie extérieure 182 est constituée par une source de courant électrique, qui peut être le réseau d'alimentation en énergie électrique du véhicule.

Le cylindre d'assistance 30 du quatrième mode de réalisation est globalement similaire à celui du deuxième mode de réalisation, représenté sur la figure 9, à la différence que le mécanisme à cames 114 du dispositif d'assistance 50 est remplacé par un actionneur électrique 186 qui agit directement sur la tige de transmission 71.

Selon la forme de réalisation représentée, la tige de transmission 71 est équipée, à son extrémité axiale amont, d'un tronçon taraudé 188 qui est monté vissé sur un arbre fileté 190 susceptible d'être entraîné en rotation autour de son axe A1 par un moteur électrique 192, qui est raccordé à la source de courant électrique 182.

Avantageusement, le moteur électrique 154 peut être commandé par une unité électronique de pilotage (non représentée sur la figure 11) de la même façon que dans le troisième mode de réalisation décrit précédemment (figure 10).

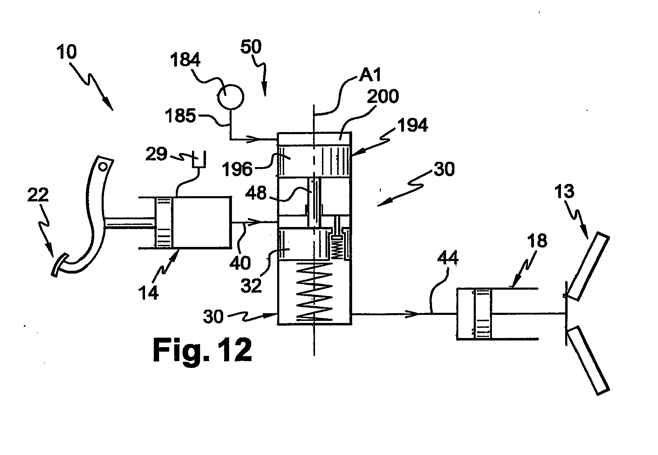

Dans le cinquième mode de réalisation, qui est illustré par les figures 12 à 19, la source d'énergie extérieure 184 est constituée par une source de pression hydraulique ou pneumatique.

Dans la suite de la description, à titre non limitatif, on considérera uniquement une source de pression hydraulique 184, bien qu'une source de pression pneumatique soit aussi envisageable.

La figure 12 illustre le principe de fonctionnement du cinquième mode de réalisation.

Dans le cinquième mode de réalisation, la tige de commande 48 du piston d'assistance 32 est liée en déplacement axial (A1) à un vérin 194 qui est raccordé à la source de pression 184 par un circuit auxiliaire de commande 185, de manière à transmettre une force d'assistance Fₐ au piston d'assistance 32 pendant la phase de débrayage.

Le vérin 194 comporte un piston 196 dit auxiliaire qui coulisse dans un cylindre auxiliaire 198 et qui délimite, en amont, une chambre auxiliaire de commande 200.

Le piston auxiliaire 196 coopère, par exemple, par contact avec la tige de commande 48 du piston d'assistance 32.

Pendant la phase de débrayage, la source de pression 184 provoque une augmentation de la pression hydraulique Pₕ dans la chambre de commande 200 du vérin 194, ce qui produit une force d'assistance Fₐ sur le piston d'assistance 32, par l'intermédiaire de la tige de commande 48, ou tige de transmission.

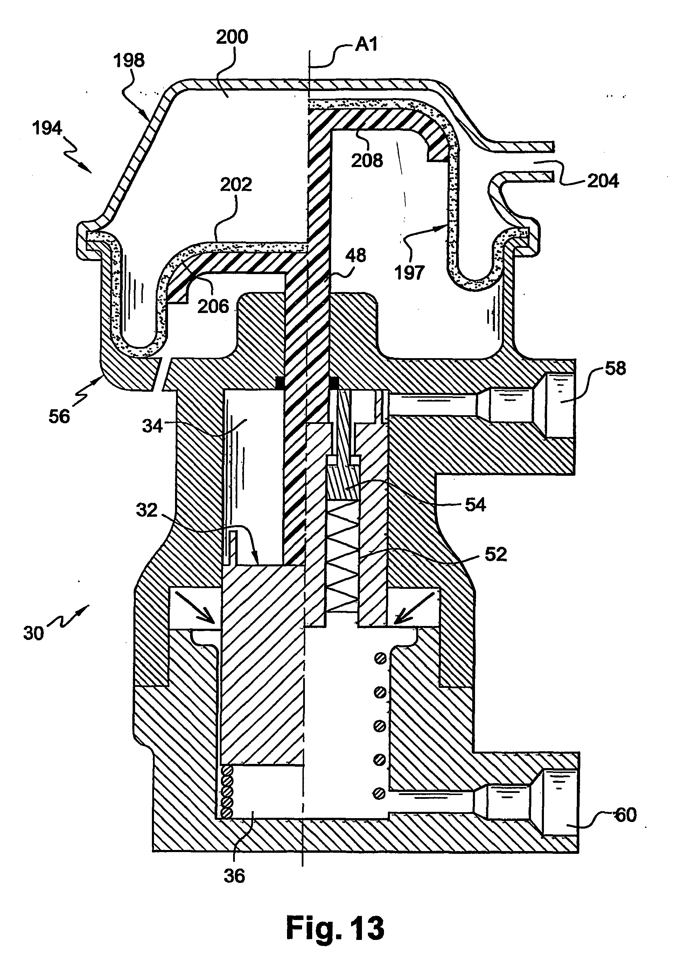

Sur la figure 13, on a représenté un exemple de cylindre d'assistance 30 équipé d'un vérin 194 conforme aux enseignements de l'invention, et dans lequel le piston auxiliaire 196 est remplacé par une membrane souple 197, ou membrane "déroulante".

Sur la partie gauche de la figure 13, le piston d'assistance 32 est représenté en position aval, et sur la partie droite, le piston d'assistance 32 est représenté en position amont.

Le piston d'assistance 32 est réalisé ici de manière similaire à celui du deuxième mode de réalisation (figure 9), à la différence que l'orifice de décharge 52 et la soupape de décharge 54 sont réalisés suivant une orientation axiale dans le corps du piston d'assistance 32, comme sur la variante décrite en référence avec la figure 4.

L'orifice de décharge 52 est décalé par rapport à l'axe pour permettre un appui centré de la tige 48 sur le piston 32. Dans le cas d'un montage incliné du cylindre d'assistance 30, l'orifice 52 est mis en position haute pour garantir la purge de l'air en aval du piston 32, lors du montage.

Le cylindre auxiliaire 198 du vérin 194 est agencé ici à l'extrémité axiale amont du cylindre d'assistance 30. Le cylindre auxiliaire 198 peut être formé dans un prolongement du corps de cylindre d'assistance 56.

La membrane 197 est scellée dans le cylindre auxiliaire de manière à délimiter, du côté de sa face transversale amont 202, la chambre de commande 200 qui est raccordée à la source de pression 184 par un orifice auxiliaire 204.

La membrane 197 est ici du type à simple effet car sa face transversale aval 206 est exposée à la pression atmosphérique.

La tige de commande 48 du piston d'assistance 32, ou tige de transmission, comporte à son extrémité axiale amont un disque d'appui 208 qui est en contact avec la face transversale aval 206 de la membrane 197.

Lorsque la pression hydraulique Pₕ augmente dans la chambre de commande 200, en dépassant la pression atmosphérique, la membrane 197 "se déroule" en exerçant une force d'appui axiale dirigée vers l'aval sur le disque 208, ce qui produit une force d'assistance Fₐ sur le piston 32, par l'intermédiaire de la tige de transmission 48.

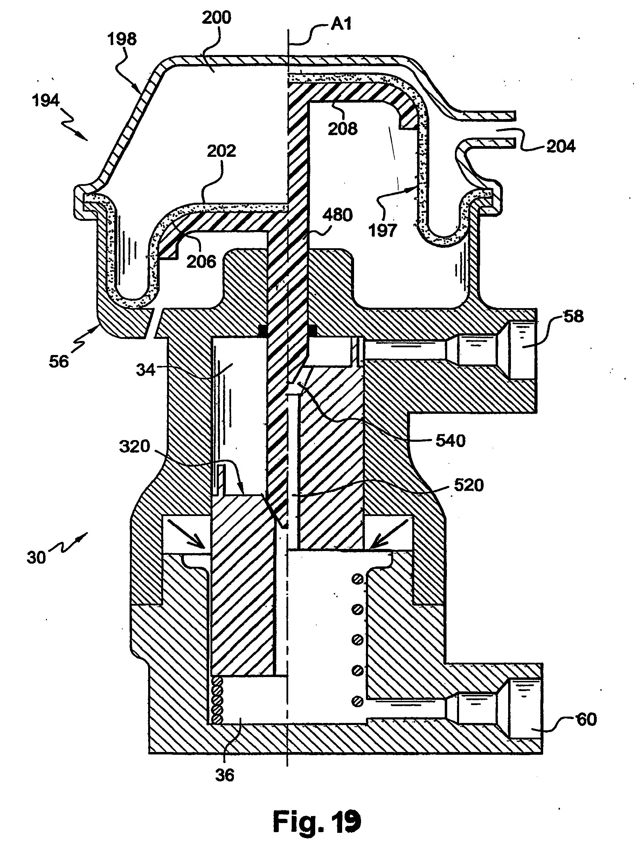

Une variante de ce dernier mode de réalisation, illustrée à la figure 19, consiste à utiliser l'appui entre la tige 480 sur le piston 320 pour commander l'ouverture d'un orifice de décharge 520. Dans ce cas, l'orifice 520 est un canal percé dans le piston 320 suivant l'axe de la tige 480, et l'extrémité de cette tige 480 a une forme complémentaire par rapport à celle du départ 540 de l'orifice 520 de manière à réaliser l'obstruction de cet orifice quand la tige est en appui sur le piston. Un joint en élastomère peu être placé entre la tige et le piston.

Quand l'embrayage est embrayé et l'assistance n'est pas activée, un petit jeu est créé entre cette tige 480 et le piston 320, l'orifice 520 est libre. Cet orifice se ferme lorsque la force d'assistance se produit. Cette variante est simple à réaliser car ne demandant pas de pièce supplémentaire, et garde tous les avantages des orifices de décharge présentés précédemment.

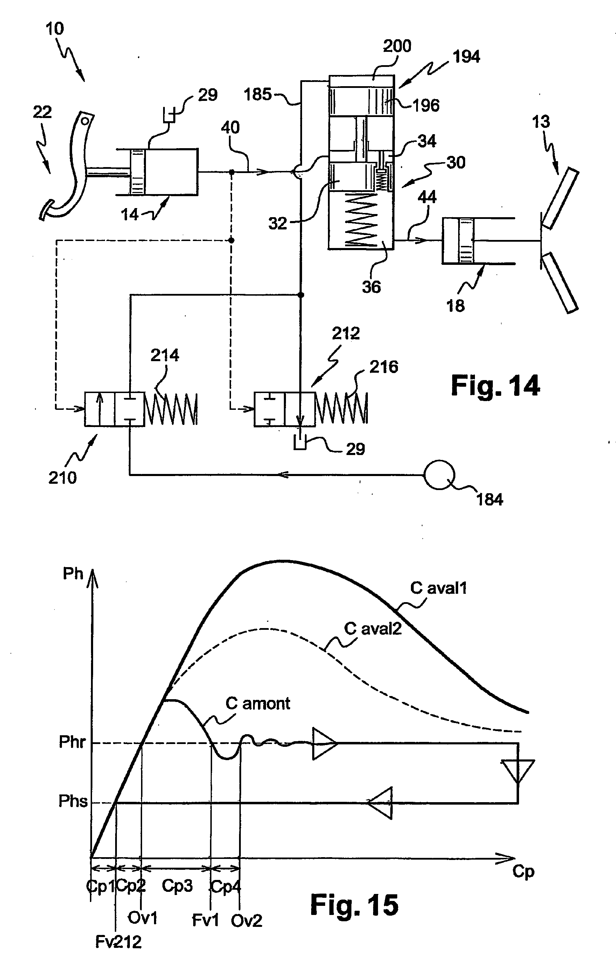

Les figures 14 à 18 illustrent plusieurs solutions possibles pour réguler la force d'assistance Fₐ produite par le vérin 194.

Selon une première solution, qui est illustrée par les figures 14 et 15, le moyen de régulation 115 de la force d'assistance Fₐ est constitué par deux distributeurs 210, 212 qui constituent respectivement une vanne de charge 210 et une vanne de décharge 212 et qui sont raccordés par le circuit auxiliaire 185 à la chambre de commande 200 du vérin 194.

En outre, la vanne de charge 210 est raccordée à la source de pression 184, et la vanne de décharge est raccordée à un réservoir de fluide 29.

La vanne de charge 210 et la vanne de décharge 212 sont commandées ici par la pression hydraulique Pₕ dans le circuit amont 40, à l'encontre de l'effort de rappel d'un ressort 214, 216 associé à chaque vanne 210, 212.

Avantageusement, la raideur du ressort 214 associé à la vanne de charge 210 est supérieure à la raideur du ressort 216 associé à la vanne de décharge 212, de manière que la l'ouverture de la vanne de charge 210 et la fermeture de la vanne de décharge 212 soient décalées dans le temps, pendant la course de débrayage.

Sur la figure 14, le système de commande 10 est représenté au repos, en position embrayée, ce qui correspond à une absence de pression dans le circuit hydraulique amont 40.

Dans cette position, la vanne de charge 210 est fermée et la vanne de décharge 212 est ouverte, de sorte que le circuit auxiliaire 185 est raccordé au réservoir 29.

On explique maintenant le fonctionnement du système de commande 10 de la figure 14, pendant la phase de débrayage, en considérant notamment la figure 15.

Sur la figure 15, la courbe Cₐᵥₐₗ₁ en trait continu représente l'évolution de la pression hydraulique Pₕ dans la chambre aval 36 du cylindre d'assistance 30, pendant la phase de débrayage, en fonction de la course Cₚ de la pédale d'embrayage 22, lorsque l'embrayage 12 est usé.

La courbe Cₐᵥₐₗ₂, en trait discontinu, représente la même évolution que la courbe Cₐᵥₐₗ₁, lorsque l'embrayage 12 est neuf.

Lorsque l'embrayage 12 s'use, la pression hydraulique Pₕ nécessaire pour effectuer l'opération de débrayage augmente.

La courbe Cornant en trait continu représente l'évolution de la pression hydraulique Pₕ dans la chambre amont 34 du cylindre d'assistance 30, pendant la phase de débrayage, en fonction de la course Cₚ de la pédale d'embrayage 22.

Lorsque la pédale d'embrayage 22 est actionnée, la pression hydraulique Pₕ augmente dans le circuit amont 40.

Après une première course Cₚ₁ de la pédale 22, la pression hydraulique Pₕ atteint une première valeur seuil Pₕₛ qui est suffisante pour provoquer le déplacement de la vanne de décharge 212 à l'encontre de son ressort 216, ce qui provoque la fermeture Fᵥ₂₁₂ de la vanne de décharge 212.

Après une seconde course Cₚ₂ de la pédale 22, la pression hydraulique Pₕ atteint une seconde valeur seuil Pₕᵣ, dite valeur régulée, qui est suffisante pour provoquer le déplacement de la vanne de charge 210 à l'encontre de son ressort 214, ce qui provoque la première ouverture Oᵥ₁ de la vanne de charge 210.

La vanne de charge 210 étant ouverte, la source de pression 184 est raccordée à la chambre de commande 200 du vérin 194, ce qui produit une force d'assistance Fₐ sur le piston 32.

La force d'assistance Fₐ appliquée sur le piston 32 provoque une diminution de la pression hydraulique Pₕ dans le circuit amont 40 de sorte que, après un laps de temps déterminé, qui correspond à une troisième course Cₚ₃ de la pédale 22, la vanne de charge 210 revient dans sa position de repos, ce qui correspond à une première fermeture Fᵥ₁ de la vanne de charge 210.

On note que la vanne de décharge 212 reste fermée car la pression hydraulique Pₕ dans le circuit amont 40 ne descend pas jusqu'à la valeur seuil Pₕₛ associée à cette vanne 212.

Le retour de la vanne de charge 210 dans sa position de repos provoque de nouveau une augmentation de la pression hydraulique Pₕ dans le circuit amont 40 puisque, la pédale 22 continuant sa course d'enfoncement, il y a baisse de pression dans la chambre de commande 200 due à la descente du vérin 196.

Après une quatrième course Cₚ₄ de la pédale 22, la pression hydraulique Pₕ atteint à nouveau la valeur régulée ce qui provoque une seconde ouverture Oᵥ₂ de la vanne de charge 210.

Cette succession d'ouvertures et de fermetures de la vanne de charge 210 se poursuit jusqu'à ce que le piston d'assistance 32 occupe sa position aval.

En pratique, pour limiter ces oscillations, on utilise une vanne de charge 210 qui s'ouvre et se ferme progressivement pour les petites oscillations à partir de la valeur régulée Pₕᵣ. De cette manière, les ouvertures et fermetures ne sont pas brutales et la position d'équilibre est atteinte plus rapidement.

La vanne de charge 210 permet donc une régulation en boucle fermée de la pression hydraulique Pₕ dans la chambre amont 34 du cylindre d'assistance 30, qui se stabilise autour de la valeur régulée Pₕᵣ.

Par conséquent, comme la pression hydraulique Pₕ dans la chambre amont 34 du cylindre d'assistance 30 est liée à la pression Pₕ dans le cylindre émetteur 14, l'effort que l'utilisateur doit appliquer sur la pédale 22 tend vers une valeur constante pendant toute la durée de la phase de débrayage.

Lorsque l'utilisateur relâche la pédale 22, la pression hydraulique Pₕ diminue dans la chambre amont 34, ce qui provoque d'abord la fermeture de la vanne de charge 210 puis, arrivé à la première valeur seuil Pₕₛ, l'ouverture de la vanne de décharge 212, permettant le retour du piston d'assistance 32 vers sa positon amont.

Un avantage de cette solution est que l'effort mis en oeuvre par l'utilisateur sur la pédale 22 n'est pas dépendant de l'usure de l'embrayage 12.

Cet avantage est illustré sur la figure 15 par le fait que la courbe de pression Cₐₘₒₙₜ est identique pour les deux courbes de pression Cₐᵥₐₗ₁, Cₐᵥₐₗ₂ associées à la chambre aval 36, celle correspondant à un embrayage 12 usé et celle correspondant à un embrayage 12 neuf.

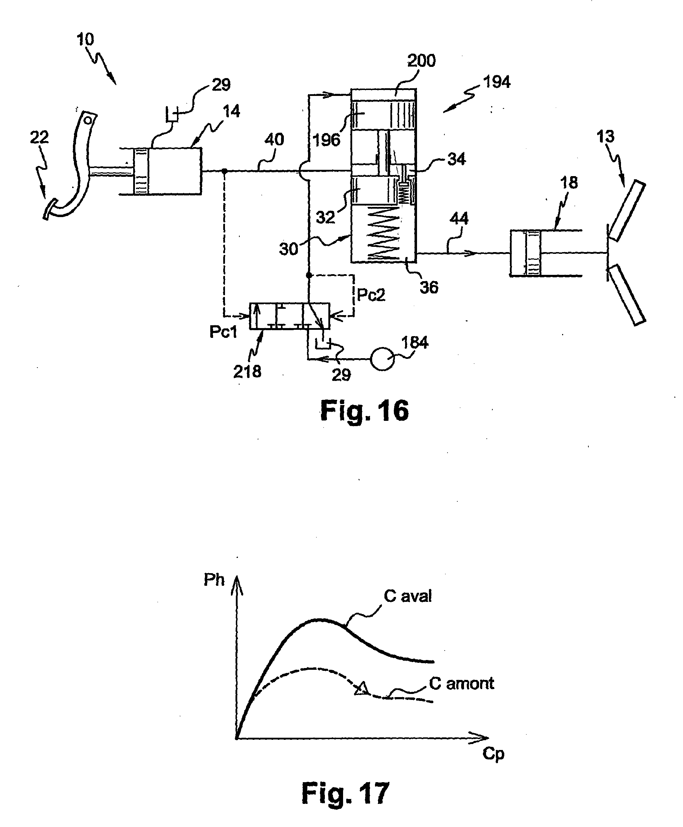

Selon une deuxième solution, qui est illustrée par les figures 16 et 17, les deux vannes 210, 212 prévues dans la première solution sont remplacées par un distributeur unique 218 à trois positions.

Une première position dite position de charge du distributeur 218 provoque le raccordement de ta chambre de commande 200 à la source de pression 184.

Une deuxième position, ou position intermédiaire, du distributeur 218 correspond à une position de fermeture du distributeur 218.

Une troisième position dite position de décharge du distributeur 218 provoque le raccordement de la chambre de commande 200 au réservoir de fluide 29.

Sur la figure 16, le distributeur 218 est représenté dans sa position de décharge.

Le distributeur 218 comporte deux pressions de commande P_{c1}, P_{c2} qui sont appliquées de part et d'autre du distributeur 218, avec des surfaces d'appui identiques.

La première pression de commande P_{c1} correspond à la pression hydraulique Pₕ dans la chambre amont 34 du cylindre d'assistance 30, et elle est appliquée du côté de la première position.

La seconde pression de commande P_{c2} correspond à la pression hydraulique Pₕ dans la chambre de commande 200 du vérin 194, et elle est appliquée du côté de la troisième position.

Le fonctionnement du distributeur 218 est le suivant.

Lorsque les deux pressions de commande P_{c1}, P_{c2} sont égales, c'est-à-dire lorsque la pression hydraulique Pₕ est égale dans la chambre amont 34 et dans la chambre de commande 200, le distributeur 218 occupe sa position intermédiaire de fermeture.

Lorsque la première pression de commande P_{c1} est supérieure à la seconde pression de commande P_{c2}, c'est-à-dire lorsque la pression dans la chambre amont 34 est supérieure à la pression dans la chambre de commande 200, le distributeur 218 occupe sa position de charge.

Lorsque la première pression de commande P_{c1} est inférieure à la seconde pression de commande P_{c2}, c'est-à-dire lorsque la pression dans la chambre amont 34 est inférieure à la pression dans la chambre de commande 200, le distributeur 218 occupe sa position de décharge.

Le mode de réalisation représenté sur la figure 16 permet ainsi de réaliser une régulation dite proportionnelle de la force d'assistance Fₐ, qui est illustrée par la figure 17.

La courbe Cₐᵥₐₗ en trait continu représente l'évolution de la pression hydraulique Pₕ dans la chambre aval 38 du cylindre d'assistance 30, pendant la phase de débrayage, en fonction de la course Cp de la pédale d'embrayage 22.

La courbe Cₐₘₒₙₜ en trait discontinu représente l'évolution de la pression hydraulique Pₕ dans la chambre amont 34 du cylindre d'assistance 30, pendant la phase de débrayage, en fonction de la course Cₚ de la pédale d'embrayage 22.

On constate que, dans le cas particulier, illustré ici, où les surfaces d'appui des pressions de commande P_{c1}, P_{c2} sont identiques, la force d'assistance Fₐ produit environ la moitié de l'effort total à fournir sur le piston du cylindre récepteur 18.

Bien entendu, on peut modifier le rapport entre la force d'assistance Fₐ et l'effort total à fournir en modifiant le rapport entre les surfaces d'appui des pressions de commande P_{c1}, P_{c2}.

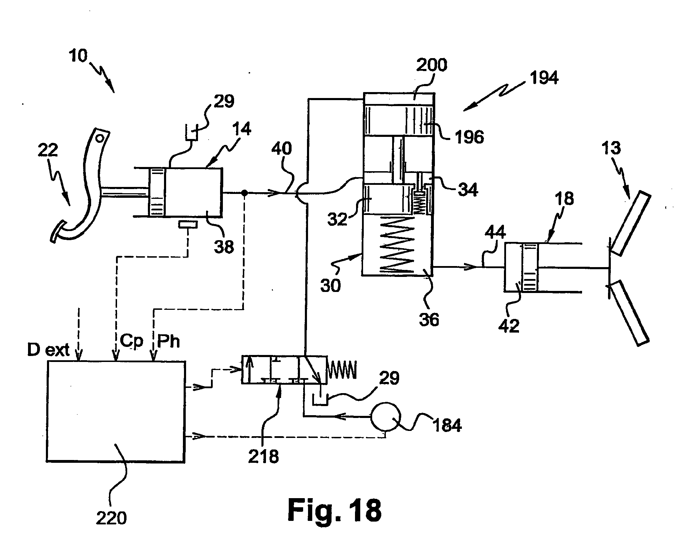

Sur la figure 18, on a représenté une troisième solution dans laquelle le moyen de régulation 115 est un distributeur à trois positions 218, comme sur la figure 16, mais qui se différencie de la deuxième solution en ce que le distributeur 218 est commandé par une unité électronique de pilotage 220.

L'unité de pilotage 220 peut commander le distributeur 218 en fonction de paramètres de commande mesurés par des capteurs tels que la course de la pédale Cₚ, la pression hydraulique dans la chambre amont 34, des données extérieures Dₑₓₜ.

L'unité de pilotage 220 peut aussi piloter la source de pression 184, ce qui permet de doser précisément la force d'assistance Fₐ désirée.

Selon le mode de réalisation représenté sur la figure 18, grâce à l'unité de pilotage 220, il est possible de choisir de manière précise la courbe d'assistance désirée. En particulier, il est possible de reproduire la courbe d'assistance de la première et de la deuxième solution (figures 15 et 17).

On note que les modes de réalisation du système de commande 10 selon l'invention, qui sont représentés avec la soupape de décharge 54 réalisée axialement dans le piston d'assistance 32 et avec un seul raccordement au réservoir de fluide 29, auraient pu être représentés avec deux raccordements au réservoir 29, comme on l'a représenté et décrit notamment en référence aux figures 2 et 3.

Selon une variante (non représentée) des différents modes de réalisation décrits ci-dessus, il est possible d'ajouter dans le passage du fluide, soit dans le circuit amont 40 ou aval 44, soit dans le circuit de commande 185 du vérin d'assistance 196, un dispositif qui réduit la section de passage du fluide dans le sens de l'embrayage.

Ce dispositif peut être un clapet qui occupe une première position formant une section de passage maximale, dans le sens du débrayage, et une seconde position formant une section de passage réduite, dans le sens de l'embrayage.

Un tel dispositif permet notamment d'éviter un choc lors d'un lâcher de pédale 22 trop rapide.

Plus généralement, le dispositif d'assistance 5 peut comporter un moyen de régulation 218 qui fait varier le valeur de la force d'assistance Fa en fonction de la pression Ph amont dans la chambre amont 34 du cylindre d'assistance 30, ou de la pression Ph aval dans la chambre aval 36, ou d'une combinaison des deux pression, selon une loi d'assistance prédéterminée.

## Revendications

1. Système de commande hydraulique (10) d'un embrayage (12), notamment de véhicule automobile, comprenant un cylindre émetteur amont (14), qui est relié par une conduite (16) à un cylindre récepteur aval (18), de manière à former un circuit hydraulique de commande (19), qui comporte un cylindre d'assistance (30) qui est interposé dans la conduite (16), entre le cylindre émetteur (14) et le cylindre récepteur (18), et qui comporte au moins un piston d'assistance (32) qui est monté coulissant axialement (A1) dans le corps (56) du cylindre d'assistance (30) entre une position amont d'embrayage et une position aval de débrayage, de manière à délimiter une chambre hydraulique amont (34) et une chambre hydraulique aval (36) de volumes variables en fonction de la position axiale du piston (32), la chambre amont (34) étant raccordée au cylindre émetteur (14) par un tronçon du circuit hydraulique dit circuit amont (40) et la chambre aval (36) étant raccordée au cylindre récepteur (18) par un tronçon du circuit hydraulique dit circuit aval (44), chaque tronçon de circuit hydraulique (40, 44) comportant un moyen (52, 102, 150) de remise à niveau du volume de fluide raccordé à au moins un réservoir de fluide (29), le cylindre d'assistance (30) comportant un dispositif d'assistance (50) qui applique une force d'assistance (Fₐ) sur le piston d'assistance (32) au cours de la phase de débrayage,
**caractérisé en ce que** le dispositif d'assistance (50) comporte un moyen de régulation (114, 115, 180, 210, 212, 218, 220) qui fait varier la valeur de la force d'assistance (Fₐ) en fonction de la course (Cₚ) de la pédale de commande de l'embrayage (22) selon une loi d'assistance prédéterminée.

2. Système de commande (10) selon l'une quelconque des revendications précédentes, **caractérisé en ce que** le dispositif d'assistance (50) comporte un organe de transmission (48, 70, 71) qui transmet la force d'assistance (Fₐ) au piston d'assistance (32).

3. Système de commande (10) selon la revendication précédente, **caractérisé en ce que** l'organe de transmission (48, 70, 71) est lié en déplacement axial au piston d'assistance (32) dans les deux sens de coulissement du piston (32).

4. Système de commande (10) selon la revendication 2, **caractérisé en ce que** l'organe de transmission (48, 71) coopère par contact avec une surface d'appui (138) associée du piston d'assistance (32) de manière que, dans le cas où la vitesse du dispositif d'assistance (50) est inférieure à la vitesse du piston d'assistance (32), le dispositif d'assistance (50) ne ralentisse pas le coulissement du piston d'assistance (32) vers l'aval.

5. Système de commande (10) selon l'une quelconque des revendications 2 à 4, **caractérisé en ce que** l'organe de transmission (48, 71) est agencé à une extrémité axiale du piston d'assistance (32).

6. Système de commande (10) selon l'une quelconque des revendications 2 à 4, **caractérisé en ce que** le piston (32) comporte un tronçon amont (62) qui délimite la chambre amont (34) et un tronçon aval (66) qui délimite la chambre aval (36), **en ce que** les deux tronçons (62, 66) sont liés en déplacement axial par une tige de liaison (70), et **en ce que** la tige de liaison (70) constitue l'organe de transmission (71) du dispositif d'assistance (50).

7. Système de commande (10) selon l'une quelconque des revendications précédentes, du type dans lequel le circuit hydraulique (19) est raccordé à un réservoir de fluide (29) en position d'embrayage, **caractérisé en ce que** le cylindre d'assistance (30) comporte au moins un orifice de décharge (52, 102, 150) qui fait communiquer au moins une chambre hydraulique (36) avec le réservoir de fluide (29), lorsque le piston d'assistance (32) occupe sa position amont, de manière à compenser les variations de volume hydraulique dans le circuit hydraulique (19) au cours du temps.

8. Système de commande (10) selon la revendication précédente, **caractérisé en ce que** l'orifice de décharge (52) est agencé dans le piston d'assistance (32) et **en ce que** l'orifice dé décharge (52) fait communiquer la chambre amont (34) avec la chambre aval (36), lorsque le piston d'assistance (32) occupe sa position amont.

9. Système de commande (10) selon la revendication 7 ou 8, **caractérisé en ce que** l'orifice de décharge (52, 150) comporte une soupape (54, 148) qui est commandée par le déplacement axial du piston d'assistance (32).

10. Système de commande (10) selon l'une quelconque des revendications précédentes, **caractérisé en ce que** le dispositif d'assistance (50) comporte un élément élastique (106, 172) qui emmagasine de l'énergie pendant la phase d'embrayage et qui restitue l'énergie au cours de la phase de débrayage pour produire la force d'assistance (Fₐ).

11. Système de commande (10) selon la revendication précédente prise en combinaison avec la revendication 1, **caractérisé en ce que** le moyen de régulation (115) est un mécanisme à cames (114) qui est entraîné par le déplacement axial du piston (32) et qui régule la force d'assistance (Fₐ) produite par l'élément élastique (106) pendant la phase de débrayage.

12. Système de commande (10) selon la revendication précédente, **caractérisé en ce que** le dispositif d'assistance (50) est logé dans le corps de cylindre (56), et **en ce que** le mécanisme à cames (114) comporte au moins une surface de commande (120, 122) qui est réalisée sur une paroi interne du corps de cylindre (56).

13. Système de commande (10) selon la revendication 11 ou 12, **caractérisé en ce que** l'élément élastique d'assistance (106) est un élément élastique de compression axiale qui est interposé axialement entre une coupelle (108) et une surface d'appui (110) fixe par rapport au corps de cylindre d'assistance (56), **en ce que** le mécanisme à cames (114) comporte au moins un galet mobile (116, 118) qui parcourt une surface de commande (120, 122) entre une position amont et une position aval correspondant respectivement aux positions amont et aval du piston d'assistance (32), et **en ce que** le galet mobile (116, 118) est lié par une première biellette (124) au piston (32) et par une seconde biellette (126) à la coupelle (108).

14. Système de commande (10) selon la revendication précédente, **caractérisé en ce que** l'axe de pivotement des biellettes (124, 126) sur le galet mobile (116, 118) est concourant à l'axe de rotation (A2) du galet (116, 118).

15. Système de commande (10) selon la revendication 13 ou 14, **caractérisé en ce que** la surface de commande (120, 122) comporte une portion amont inclinée (134) par rapport à l'axe de coulissement (A1), et une portion aval (136) globalement parallèle à l'axe de coulissement (A1) de manière que, au cours d'une première partie de la phase de débrayage, le galet mobile (116, 118) se déplace d'abord sur la portion inclinée (134) vers l'axe (A1) et vers l'aval, à partir de sa position amont, en transmettant une partie de l'effort de détente de l'élément élastique d'assistance (106) au piston d'assistance (32), par un effet de démultiplication, puis au cours d'une deuxième partie de la phase de débrayage, le galet mobile (116, 118) se déplace sur la portion aval (136) vers l'aval, suivant une direction globalement axiale, en transmettant la totalité de l'effort de détente de l'élément élastique d'assistance (106) au piston d'assistance (32).

16. Système de commande (10) selon la revendication précédente, **caractérisé en ce que** la distance entre les axes de pivotement de la seconde biellette (126) est telle que, en position amont du galet mobile (116, 118), le galet dépasse vers l'amont le point (B1) de la surface de commande (120, 122) où la seconde biellette (126) est perpendiculaire à la surface de commande (120, 122), de manière que l'effort de détente de l'élément élastique d'assistance (106) sollicite le galet mobile (116, 118) vers sa position amont.

17. Système de commande (10) selon l'une quelconque des revendications 13 à 16, **caractérisé en ce que** la dimension axiale de l'élément élastique d'assistance (106) à l'état détendu est inférieure à la distance axiale entre la coupelle (108) et la surface d'appui fixe (110) associée, lorsque le piston (32) occupe sa position aval, de manière à supprimer la force d'assistance (Fₐ) pendant la fin de la course du piston (32) vers l'aval.

18. Système de commande (10) selon la revendication 10, **caractérisé en ce que** le dispositif d'assistance (50) comporte un actionneur électrique (170) qui commande la détente de l'élément élastique (172) pendant la phase de débrayage.

19. Système de commande (10) selon la revendication précédente prise en combinaison avec la revendication 1, **caractérisé en ce que** le moyen de régulation (115) du dispositif d'assistance (50) est une unité électronique de pilotage (180) qui commande l'actionneur électrique (170).

20. Système de commande (10) selon l'une quelconque des revendications 10 à 19, **caractérisé en ce que** l'élément élastique d'assistance (106, 172) est un ressort hélicoïdal de compression.

21. Système de commande (10) selon l'une quelconque des revendications 1 à 9, **caractérisé en ce que** le dispositif d'assistance (50) est raccordé à une source d'énergie qui est externe au système de commande (10) et qui est embarquée dans le véhicule que le système de commande (10) équipe, et **en ce que** ladite énergie produit la force d'assistance (Fₐ) qui est transmise au piston (32).

22. Système de commande (10) selon la revendication précédente, **caractérisé en ce que** le dispositif d'assistance (50) comporte un actionneur électrique (186) qui est commandé de manière à transmettre une force d'assistance (Fₐ) au piston (32) pendant la phase de débrayage.

23. Système de commande (10) selon la revendication précédente prise en combinaison avec la revendication 1, **caractérisé en ce que** le moyen de régulation (115) du dispositif d'assistance (50) est une unité électronique de pilotage qui commande l'actionneur électrique (186) produisant la force d'assistance (Fₐ).

24. Système de commande (10) selon la revendication 21, **caractérisé en ce que** le dispositif d'assistance (50) comporte un vérin (194) qui est raccordé à une source de pression (184) hydraulique ou pneumatique et qui transmet une force d'assistance (Fₐ) au piston (32) pendant la phase de débrayage.

25. Système de commande (10) selon la revendication précédente prise en combinaison avec la revendication 1, **caractérisé en ce que** le moyen de régulation (115) du dispositif d'assistance (50) comporte au moins un distributeur (210, 212, 218) qui est interposé entre le vérin (194) et la source de pression (184) hydraulique ou pneumatique.

26. Système de commande (10) selon la revendication précédente, **caractérisé en ce que** le moyen de régulation (115) comporte un distributeur (210) à deux positions qui est raccordé à une source de pression (184) pour former une vanne de charge et un distributeur (212) à deux positions qui est raccordé à un réservoir de fluide (29) pour former une vanne de décharge, et **en ce que** chaque distributeur (210, 212) est commandé par la pression hydraulique (Pₕ) dans le circuit amont (40), de manière que la pression hydraulique (Pₕ) dans le circuit amont (40) tende vers une première valeur constante (Pₕᵣ) lors d'une course de débrayage, et tende vers une seconde valeur constante (Pₕₛ), inférieure à la première valeur (Pₕᵣ), lors d'une course d'embrayage.

27. Système de commande (10) selon la revendication 26, **caractérisé en ce que** le moyen de régulation (115) comporte un distributeur (218) à trois positions, une position de charge qui est raccordée à une source de pression (184), une position intermédiaire de fermeture, une position de décharge qui est raccordée à un réservoir de fluide (29), et **en ce que** le distributeur (218) est commandé, du côté de la position de charge, par la pression hydraulique (Pₕ) dans le circuit amont (40), et du côté de la position de décharge par la pression hydraulique dans la chambre de commande (200) du vérin (194), de manière que la force d'assistance (Fₐ) appliquée sur le piston d'assistance (32) pendant la phase de débrayage soit proportionnelle à la pression hydraulique (Pₕ) dans le circuit aval (44).

28. Système de commande (10) selon la revendication 25, **caractérisé en ce que** le distributeur (218) est commandé par une unité électronique de pilotage (220).

29. Système de commande (10) selon l'une quelconque des revendications précédentes, **caractérisé en ce que** le piston (32) comporte au moins un élément élastique (46, 106) qui rappelle le piston (32) vers sa position amont.

30. Système de commande suivant l'une quelconque des revendications précédentes, **caractérisé en ce que** le dispositif d'assistance (5) comporte un moyen de régulation (218) qui fait varier le valeur de la force d'assistance (Fa) en fonction de la pression Ph amont dans la chambre amont (34) du cylindre d'assistance (30), ou de la pression Ph aval dans la chambre aval (36), ou d'une combinaison des deux pressions selon une loi d'assistance prédéterminé.

31. Système de commande suivant l'une quelconque des revendications précédentes, **caractérisé en ce que** l'orifice (520) est un canal percé dans le piston (320) suivant l'axe de la tige (480) et l'extrémité de cette tige (480) a une forme complémentaire par rapport à celle du départ de l'orifice (520) de manière à réaliser l'obstruction de cet orifice quand la tige (480) est en appui sur le piston.

## Claims

1. Hydraulic control system (10) for a clutch (12), in particular for a motor vehicle, comprising an upstream sending cylinder (14) connected by a conduit (16) to a downstream receiving cylinder (18), so as to form a hydraulic control circuit (19), which comprises an assistance cylinder (30) that is interposed in the conduit (16), between the sending cylinder (14) and the receiving cylinder (18), and which comprises at least one assistance piston (32) that is mounted so as to slide axially (A1) in the body (56) of the assistance cylinder (30) between an upstream engagement position and a downstream disengagement position, so as to delimit an upstream hydraulic chamber (34) and a downstream hydraulic chamber (36) with variable volumes according to the axial position of the piston (32), the upstream chamber (34) being connected to the sending cylinder (14) by a portion of the hydraulic circuit referred to as the upstream circuit (40) and the downstream chamber (36) being connected to the receiving cylinder (18) by a portion of the hydraulic circuit referred to as the downstream circuit (44), each hydraulic circuit portion (40, 44) comprising a means (52, 102, 150) of relevelling the volume of fluid connected to at least one fluid reservoir (29), the assistance cylinder (30) comprising an assistance device (50) that applies an assistance force (Fₐ) to the assistance piston (32) during the disengagement phase, **characterised in that** the assistance device (50) comprises a regulation means (114, 115, 180, 210, 212, 218, 220) which varies the value of the assistance force (Fₐ) according to the travel (C_{P}) of the clutch control pedal (22) in accordance with a predetermined assistance law.

2. Control system (10) according to any one of the preceding claims, **characterised in that** the assistance device (50) comprises a transmission member (48, 70, 71) which transmits the assistance force (Fₐ) to the assistance piston (32).

3. Control system (10) according to the preceding claim, **characterised in that** the transmission member (48, 70, 71) is connected in terms of axial movement to the assistance piston (32) in both directions of sliding of the piston (32).

4. Control system (10) according to claim 2, **characterised in that** the transmission member (48, 71) cooperates by contact with an associated abutment surface (138) of the assistance piston (32) so that, in the case where the speed of the assistance device (50) is less than the speed of the assistance piston (32), the assistance device (50) does not slow down the sliding of the assistance piston (32) towards the downstream end.

5. Control system (10) according to any one of claims 2 to 4, **characterised in that** the transmission member (48, 71) is arranged at an axial end of the assistance piston (32).

6. Control system (10) according to any one of claims 2 to 4, **characterised in that** the piston (32) comprises an upstream portion (62) that delimits the upstream chamber (34) and a downstream portion (66) that delimits the downstream chamber (36), **in that** the two portions (62, 66) are connected in axial movement by a connecting rod (70), and **in that** the connecting rod (70) constitutes the transmission member (71) of the assistance device (50).

7. Control system (10) according to any one of the preceding claims, of the type in which the hydraulic circuit (19) is connected to a fluid reservoir (29) in the engagement position, **characterised in that** the assistance cylinder (30) comprises at least one discharge orifice (52, 102, 150) which makes at least one hydraulic chamber (36) communicate with the fluid reservoir (29), when the assistance piston (32) is occupying its upstream position, so as to compensate for the variations in hydraulic volume in the hydraulic circuit (19) over time.

8. Control system (10) according to the preceding claim, **characterised in that** the discharge orifice (52) is arranged in the assistance piston (32), and **in that** the discharge orifice (52) makes the upstream chamber (34) communicate with the downstream chamber (36), when the assistance piston (32) is occupying its upstream position.

9. Control system (10) according to claim 7 or 8, **characterised in that** the discharge orifice (52, 150) comprises a valve (54, 148) that is controlled by the axial movement of the assistance piston (32).

10. Control system (10) according to any one of the preceding claims, **characterised in that** the assistance device (50) comprises an elastic element (106, 172) which stores energy during the engagement phase and which restores the energy during the disengagement phase in order to produce the assistance force (Fₐ).

11. Control system (10) according to the preceding claim taken in combination with claim 1, **characterised in that** the regulation means (115) is a cam mechanism (114) which is driven by the axial movement of the piston (32) and which regulates the assistance force (Fₐ) produced by the elastic element (106) during the disengagement phase.

12. Control system (10) according to the preceding claim, **characterised in that** the assistance device (50) is housed in the cylinder body (56), and **in that** the cam mechanism (114) comprises at least one control surface (120, 122) that is produced on an internal wall of the cylinder body (56).

13. Control system (10) according to claim 11 or 12, **characterised in that** the elastic assistance element (106) is an axial compression elastic element that is interposed axially between a cup (108) and an abutment surface (110) fixed with respect to the assistance cylinder body (56), **in that** the cam mechanism (114) comprises at least one movable roller (116, 118) which travels over a control surface (120, 122) between an upstream position and a downstream position corresponding respectively to the upstream and downstream positions of the assistance piston (32), and **in that** the movable roller (116, 118) is connected by a first connecting rod (124) to the piston (32) and by a second connecting rod (126) to the cup (108).

14. Control system (10) according to the preceding claim, **characterised in that** the axis by which the connecting rods (124, 126) pivot on the movable roller (116, 118) is concurrent with the rotation axis (A2) of the roller (116, 118).

15. Control system (10) according to claim 13 or 14, **characterised in that** the control surface (120, 122) comprises an upstream portion (134) inclined with respect to the sliding axis (A1), and a downstream portion (136) roughly parallel to the sliding axis (A1) so that, during a first part of the disengagement phase, the movable roller (116, 118) moves first of all on the inclined portion (134) towards the axis (A1) and in the downstream direction, from its upstream position, transmitting part of the relaxation force of the elastic assistance element (106) to the assistance piston (32), by a step-down effect, and then, during a second part of the disengagement phase, the movable roller (116, 118) moves on the downstream portion (136) in the downstream direction, in a roughly axial direction, transmitting all the relaxation force of the elastic assistance element (106) to the assistance piston (32).

16. Control system (10) according to the preceding claim, **characterised in that** the distance between the pivot axes of the second connecting rod (126) is such that, in the upstream position of the movable roller (116, 118), the roller moves in the upstream direction beyond the point (B1) on the control surface (120, 122) where the second connecting rod (126) is perpendicular to the control surface (120, 122), so that the relaxation force of the elastic assistance element (106) biases the movable roller (116, 118) towards its upstream position.

17. Control system (10) according to any one of claims 13 to 16, **characterised in that** the axial dimension of the elastic assistance element (106) in the relaxed state is less than the axial distance between the cup (108) and the associated fixed abutment surface (110), when the piston (32) occupies its downstream position, so as to eliminate the assistance force (Fₐ) during the end of the travel of the piston (32) in the downstream direction.

18. Control system (10) according to claim 10, **characterised in that** the assistance device (50) comprises an electrical actuator (170) that controls the relaxation of the elastic element (172) during the disengagement phase.

19. Control system (10) according to the preceding claim taken in combination with claim 1, **characterised in that** the means (115) of regulating the assistance device (50) is an electronic control unit (180) that controls the electrical actuator (170).

20. Control system (10) according to any one of claims 10 to 19, **characterised in that** the elastic assistance element (106, 172) is a helical compression spring.

21. Control system (10) according to any one of claims 1 to 9, **characterised in that** the assistance device (50) is connected to an energy source that is external to the control system (10) and that is installed in the vehicle that the control system (10) equips, and **in that** the said energy produces the assistance force (Fₐ) that is transmitted to the piston (32).

22. Control system (10) according to the preceding claim, **characterised in that** the assistance device (50) comprises an electrical actuator (186) controlled so as to transmit an assistance force (Fₐ) to the piston (32) during the disengagement phase.

23. Control system (10) according to the preceding claim taken in combination with claim 1, **characterised in that** the means (115) of regulating the assistance device (50) is an electronic control unit that controls the electrical actuator (186) producing the assistance force (Fₐ) .

24. Control system (10) according to claim 21, **characterised in that** the assistance device (50) comprises a ram (194) that is connected to a hydraulic or pneumatic pressure source (184) and that transmits an assistance force (Fₐ) to the piston (32) during the disengagement phase.

25. Control system (10) according to the preceding claim taken in combination with claim 1, **characterised in that** the means (115) of regulating the assistance device (50) comprises at least one control valve (210, 212, 218) interposed between the ram (194) and the hydraulic or pneumatic pressure source (184).

26. Control system (10) according to the preceding claim, **characterised in that** the regulation means (115) comprises a two-position control valve (210) connected to a pressure source (184) in order to form a charging valve and a two-position control valve (212) connected to a fluid reservoir (29) in order to form a discharge valve, and **in that** each control valve (210, 212) is controlled by the hydraulic pressure (Pₕ) in the upstream circuit (40), so that the hydraulic pressure (Pₕ) in the upstream circuit (40) tends towards a first constant value (Pₕᵣ) during a disengagement travel and tends towards a second constant value (Pₕₛ), less than the first value (Pₕᵣ), during an engagement travel.

27. Control system (10) according to claim 26, **characterised in that** the regulation means (115) comprises a three-position control valve (218) which has a charging position connected to a pressure source (184), an intermediate closure position, and a discharge position connected to a fluid reservoir (29), and **in that** the control valve (218) is controlled on the charging position side by the hydraulic pressure (Pₕ) in the upstream circuit (40), and on the discharge position side by the hydraulic pressure in the control chamber (200) of the ram (194), so that the assistance force (Fₐ) applied to the assistance piston (32) during the disengagement phase is proportional to the hydraulic pressure (Pₕ) in the downstream circuit (44).

28. Control system (10) according to claim 25, **characterised in that** the distributor (218) is controlled by an electronic control unit (220).

29. Control system (10) according to any one of the preceding claims, **characterised in that** the piston (32) comprises at least one elastic element (46, 106) that returns the piston (32) towards its upstream position.

30. Control system according to any one of the preceding claims, **characterised in that** the assistance device (5) comprises a regulation means (218) which varies the value of the assistance force (Fₐ) according to the upstream pressure Ph in the upstream chamber (34) of the assistance cylinder (30) or the downstream pressure Ph in the downstream chamber (36), or a combination of the two pressures, in accordance with a predetermined assistance law.

31. Control system according to any one of the preceding claims, **characterised in that** the orifice (520) is a channel pierced in the piston (320) along the axis of the rod (480) and the end of this rod (480) has a complementary shape with respect to that of the start of the orifice (520) so as to obstruct this orifice when the rod (480) is in abutment against the piston.

## Patentansprüche

1. Hydraulisches Betätigungssystem (10) für eine Kupplung (12), insbesondere für ein Kraftfahrzeug, mit einem flussaufwärts gelagerten Geberzylinder (14), der durch eine Leitung (16) mit einem flussabwärts gelagerten Nehmerzylinder (18) verbunden ist, um eine hydraulische Betätigungsschaltung (19) zu bilden, die einen zwischen dem Geberzylinder (14) und dem Nehmerzylinder (18) in die Leitung (16) eingesetzten Unterstützungszylinder (30) umfasst, der mindestens einen Unterstützungskolben (32) aufweist, welcher im Gehäuse (56) des Unterstützungszylinders (30) zwischen einer flussaufwärts gelagerten Kupplungsposition und einer flussabwärts gelagerten Entkupplungsposition axial (A1) beweglich gelagert ist, um eine flussaufwärts gelagerte hydraulische Kammer (34) und eine flussabwärts gelagerte hydraulische Kammer (36) mit variablen Volumina, die von der axialen Positionierung des Kolbens (32) abhängen, zu begrenzen, wobei die flussaufwärts gelagerte Kammer (34) an den Geberzylinder (14) über einen als flussaufwärts gelagerte Schaltung (40) bezeichneten Abschnitt der hydraulischen Schaltung und die flussabwärts gelagerte Kammer (36) an den Nehmerzylinder (18) über einen als flussabwärts gelagerte Schaltung (44) bezeichneten Abschnitt der hydraulischen Schaltung angeschlossen ist, wobei jeder Abschnitt (40, 44) der hydraulischen Schaltung ein Mittel (52, 102, 150) zur Regulierung des Flüssigkeitsvolumens aufweist, welches an mindestens ein Fluidreservoir (29) angeschlossen ist, wobei der Unterstützungszylinder (30) eine Unterstützungsvorrichtung (50) aufweist, die im Verlauf der Auskuppelphase eine Unterstützungskraft (Fₐ) auf den Unterstützungskolben (32) ausübt,
**dadurch gekennzeichnet, dass** die Unterstützungsvorrichtung (50) ein Regelmittel (114, 115, 180, 210, 212, 218, 220) umfasst, welches den Wert der Unterstützungskraft (Fₐ) in Abhängigkeit vom Weg (Cp) des Kupplungsbetätigungspedals (22) entsprechend eines vorbestimmten Unterstützungsgesetzes variiert.

2. Betätigungssystem (10) nach dem vorherigen Anspruch, **dadurch gekennzeichnet, dass** die Unterstützungsvorrichtung (50) ein Übertragungsorgan (48, 70, 71) umfasst, welches die Unterstützungskraft (Fₐ) auf den Unterstützungskolben (32) überträgt.

3. Betätigungssystem (10) nach dem vorherigen Anspruch, **dadurch gekennzeichnet, dass** das Übertragungsorgan (48, 70, 71) axial verschiebefest mit dem Unterstützungskolben (32) in den beiden Verschieberichtungen des Kolbens (32) verbunden ist.

4. Betätigungssystem (10) nach Anspruch 2, **dadurch gekennzeichnet, dass** das Übertragungsorgan (48, 71) durch Kontakt mit einer mit dem Unterstützungskolben (32) verbundenen Auflagefläche (138) derart zusammenwirkt, dass in dem Fall, wo die Geschwindigkeit der Unterstützungsvorrichtung (50) geringer ist als die Geschwindigkeit des Unterstützungskolbens (32), die Unterstützungsvorrichtung (50) die flussabwärts gerichtete Verschiebung des Unterstützungskolbens (32) nicht verlangsamt.

5. Betätigungssystem (10) nach einem der Ansprüche 2 bis 4, **dadurch gekennzeichnet, dass** das Übertragungsorgan (48, 71) an einem axialen Ende des Unterstützungskolbens (32) angeordnet ist

6. Betätigungssystem (10) nach einem der Ansprüche 2 bis 4, **dadurch gekennzeichnet, dass** der Kolben (32) ein flussaufwärts gelagertes Teilstück (62), welches die flussaufwärts gelagerte Kammer (34) begrenzt, und ein flussabwärts gelagertes Teilstück (66), welches die flussabwärts gelagerte Kammer (36) begrenzt, umfasst, und dass die beiden Teilstücke (62, 66) durch eine Verbindungsstange (70) axial verschiebefest miteinander verbunden sind, und dass die Verbindungsstange (70) das Übertragungsorgan (71) der Unterstützungsvorrichtung (50) bildet.

7. Betätigungssystem (10) nach einem der vorherigen Ansprüche, bei dem die hydraulische Schaltung (19) in der eingekuppelten Position an ein Fluidreservoir (29) angeschlossen ist, **dadurch gekennzeichnet, dass** der Unterstützungszylinder (30) mindestens eine Entlastungsöffnung (52, 102, 150) aufweist, durch die mindestens eine hydraulische Kammer (36) mit dem Fluidreservoir (29) kommuniziert, wenn der Unterstützungskolben (32) seine flussaufwärts gelagerte Position einnimmt, um die Veränderungen des hydraulischen Volumens in der hydraulischen Schaltung (19) im Lauf der Zeit zu kompensieren.

8. Betätigungssystem (10) nach dem vorherigen Anspruch, **dadurch gekennzeichnet, dass** die Entlastungsöffnung (52) in dem Unterstützungskolben (32) angeordnet ist, und dass durch die Entlastungsöffnung (52) die flussaufwärts gelagerte Kammer (34) mit der flussabwärts gelagerten Kammer (36) kommuniziert, wenn der Unterstützungskolben (32) seine flussaufwärts gelagerte Position einnimmt.

9. Betätigungssystem (10) nach Anspruch 7 oder 8, **dadurch gekennzeichnet, dass** die Entlastungsöffnung (52, 150) ein Ventil (54, 148) umfasst, welches durch die axiale Verschiebung des Unterstützungskolbens (32) betätigt wird.

10. Betätigungssystem (10) nach einem der vorherigen Ansprüche, **dadurch gekennzeichnet, dass** die Unterstützungsvorrichtung (50) ein elastisches Element (106, 172) umfasst, welches während der Einkuppelphase Energie speichert und welches die Energie im Laufe der Auskuppelphase wieder abgibt um die Unterstützungskraft (Fₐ) zu erzeugen.

11. Betätigungssystem (10) nach dem vorherigen Anspruch in Kombination mit Anspruch 1, **dadurch gekennzeichnet, dass** das Regelmittel (115) ein Nockenmechanismus (114) ist, der durch die axiale Verschiebung des Kolbens (32) angetrieben wird, und der die vom elastischen Element (106) in der Auskuppelphase erzeugte Unterstützungskraft (Fₐ) regelt.

12. Betätigungssystem (10) nach dem vorherigen Anspruch, **dadurch gekennzeichnet, dass** die Unterstützungsvorrichtung (50) in dem Zylinderkörper (56) aufgenommen ist, und dass der Nockenmechanismus (114) mindestens eine Betätigungsfläche (120, 122) aufweist, die auf einer inneren Wand des Zylinderkörpers (56) ausgeführt ist.

13. Betätigungssystem (10) nach Anspruch 11 oder 12, **dadurch gekennzeichnet, dass** das elastische Unterstützungselement (106) ein axial zusammendrückbares elastisches Element ist, welches axial zwischen eine Schale (108) und eine relativ zum Körper (56) des Unterstützungszylinders feste Auflagefläche (110) eingesetzt ist, und dass der Nockenmechanismus (114) mindestens eine bewegliche Laufrolle (116, 118) aufweist, die entlang einer Betätigungsfläche (120, 122) zwischen einer flussaufwärts gelagerten Position und einer flussabwärts gelagerten Position läuft, welche jeweils der flussaufwärts gelagerten und flussabwärts gelagerten Position des Unterstützungskolbens (32) entsprechen, und dass die bewegliche Laufrolle (116, 118) durch eine erste Pleuelstange (124) mit dem Kolben (32) und durch eine zweite Pleuelstange (126) mit der Schale (108) verbunden ist.

14. Betätigungssystem (10) nach dem vorherigen Anspruch, **dadurch gekennzeichnet, dass** die Drehachse der Pleuelstangen (124, 126) auf der beweglichen Laufrolle (116, 118) mit der Rotationsachse (A2) der Laufrolle (116, 118) zusammenfällt.

15. Betätigungssystem (10) nach Anspruch 13 oder 14, **dadurch gekennzeichnet, dass** die Betätigungsfläche (120, 122) einen flussaufwärts gelagerten zur Verschiebeachse (A1) geneigten Bereich (134) und einen im wesentlichen parallel zur Verschiebeachse (A1) verlaufenden flussabwärts gelagerten Bereich (136) derart umfasst, dass im Verlauf eines ersten Teils der Auskuppelphase die bewegliche Laufrolle (116, 118) sich ausgehend von ihrer flussaufwärts gelagerten Position zunächst auf dem geneigten Bereich (134) zur Achse (A1) und flussabwärts verlagert, wobei sie einen Teil der Kraft der Entspannung des elastischen Unterstützungselements (106) durch einen Untersetzungseffekt auf den Unterstützungskolben (32) überträgt, und dann im Verlauf eines zweiten Teils der Auskuppelphase die bewegliche Laufrolle (116, 118) sich auf dem flussabwärts gelagerten Bereich (136) flussabwärts verlagert, wobei sie einer im wesentlichen axialen Richtung folgt und die Gesamtheit der Kraft der Entspannung des elastischen Unterstützungselements (106) auf den Unterstützungskolben (32) überträgt.

16. Betätigungssystem (10) nach dem vorherigen Anspruch, **dadurch gekennzeichnet, dass** die Entfernung zwischen den Drehachsen der zweiten Pleuelstange (126) derart ist, dass in der flussaufwärts gelagerten Position der bewegliche Laufrolle (116, 118) die Laufrolle an dem Punkt (B1) der Betätigungsfläche (120, 122) flussaufwärts vorbeizieht, wo die zweite Pleuelstange (126) derart senkrecht zur Betätigungsfläche (120, 122) ist, dass die Kraft der Entspannung des elastischen Unterstützungselements (106) die bewegliche Laufrolle (116, 118) in ihre flussaufwärts gelagerte Position beaufschlagt.

17. Betätigungssystem (10) nach einem der Ansprüche 13 bis 16, **dadurch gekennzeichnet, dass** die axiale Abmessung des elastischen Unterstützungselements (106) im entspannten Zustand kleiner ist als die axiale Entfernung zwischen der Schale (108) und der zugeordneten festen Auflagefläche (110), wenn der Kolben (32) seine flussabwärts gelagerte Position einnimmt, um die Unterstützungskraft (Fₐ) während des Endes des Weges des Kolbens (32) zu seiner flussabwärts gelagerten Position aufzuheben.

18. Betätigungssystem (10) nach Anspruch 10, **dadurch gekennzeichnet, dass** die Unterstützungsvorrichtung (50) eine elektrische Betätigungseinrichtung (170) umfasst, welche die Entspannung des elastischen Elements (172) während der Auskuppelphase steuert.

19. Betätigungssystem (10) nach dem vorherigen Anspruch in Kombination mit Anspruch 1, **dadurch gekennzeichnet, dass** das Regelmittel (115) der Unterstützungsvorrichtung (50) eine elektronische Steuereinheit (180) ist, welche die elektrische Betätigungseinrichtung (170) steuert.

20. Betätigungssystem (10) nach einem der Ansprüche 10 bis 19, **dadurch gekennzeichnet, dass** das elastische Unterstützungselement (106, 172) eine Schraubendruckfeder ist.

21. Betätigungssystem (10) nach einem der Ansprüche 1 bis 9, **dadurch gekennzeichnet, dass** die Unterstützungsvorrichtung (50) an eine Energiequelle angeschlossen ist, die außerhalb des Betätigungssystems (10) ist und die wie das Betätigungssystem (10) in das damit ausgerüstete Fahrzeug eingebracht ist, und dass die besagte Energie die an den Kolben (32) übertragene Unterstützungskraft (Fₐ) hervorruft.

22. Betätigungssystem (10) nach dem vorherigen Anspruch, **dadurch gekennzeichnet, dass** die Unterstützungsvorrichtung (50) eine elektrische Betätigungseinrichtung (186) umfasst, die betätigt wird, um während der Auskuppelphase eine Unterstützungskraft (Fₐ) auf den Kolben (32) zu übertragen.

23. Betätigungssystem (10) nach dem vorherigen Anspruch in Kombination mit Anspruch 1, **dadurch gekennzeichnet, dass** das Regelmittel (115) der Unterstützungsvorrichtung (50) eine elektronische Steuereinheit ist, welche die elektrische Betätigungseinrichtung (186) steuert, wobei die Unterstützungskraft (Fₐ) hervorgerufen wird.

24. Betätigungssystem (10) nach Anspruch 21, **dadurch gekennzeichnet, dass** die Unterstützungsvorrichtung (50) einen Zylinder (194) umfasst, der an eine hydraulische oder pneumatische Druckquelle (184) angeschlossen ist und der während der Auskuppelphase eine Unterstützungskraft (Fₐ) auf den Kolben (32) überträgt.

25. Betätigungssystem (10) nach dem vorherigen Anspruch in Kombination mit Anspruch 1, **dadurch gekennzeichnet, dass** das Regelmittel (115) der Unterstützungsvorrichtung (50) mindestens einen Verteiler (210, 212, 218) umfast, welcher zwischen den Zylinder (194) und die hydraulische oder pneumatische Druckquelle (184) eingesetzt ist.

26. Betätigungssystem (10) nach dem vorherigen Anspruch, **dadurch gekennzeichnet, dass** das Regelmittel (115) einen Verteiler (210) mit zwei Positionen, welcher an eine Druckquelle (184) angeschlossen ist, um einen Belastungsschieber zu bilden, und einen Verteiler (212) mit zwei Positionen umfast, welcher an ein Fluidreservoir (29) angeschlossen ist, um einen Entlastungsschieber zu bilden, und dass jeder Verteiler (210, 212) durch den hydraulischen Druck (Pₕ) in der flussaufwärts gelagerten Schaltung (40) derart betätigt wird, dass der hydraulische Druck (Pₕ) in der flussaufwärts gelagerten Schaltung (40) bei einem Auskuppelweg gegen einen ersten konstanten Wert (Pₕᵣ) strebt, und bei einem Einkuppelweg gegen einen zweiten konstanten Wert (Pₕₛ) strebt, welcher geringer ist als der erste Wert (Pₕᵣ).

27. Betätigungssystem (10) nach Anspruch 26, **dadurch gekennzeichnet, dass** das Regelmittel (115) einen Verteiler (218) mit drei Positionen, einer Belastungsposition, die an eine Druckquelle (184) angeschlossen ist, einer mittleren Verschlussposition und einer Entlastungsposition, die an ein Fluidreservoir (29) angeschlossen ist, umfasst, und dass der Verteiler (218) von der Seite der Belastungsposition durch den hydraulischen Druck (Pₕ) in der flussaufwärts gelagerten Schaltung (40) und von der Seite der Entlastungsposition durch den hydraulischen Druck in der Betätigungskammer (200) des Zylinders (194) derart betätigt wird, dass die während der Auskuppelphase auf den Unterstützungskolben (32) ausgeübte Unterstützungskraft (Fₐ) proportional zum hydraulischen Druck (Pₕ) in der flussabwärts gelagerten Schaltung (44) ist.

28. Betätigungssystem (10) nach Anspruch 25, **dadurch gekennzeichnet, dass** der Verteiler (218) durch eine elektronische Steuereinheit gesteuert wird.

29. Betätigungssystem (10) nach einem der vorherigen Ansprüche, **dadurch gekennzeichnet, dass** der Kolben (32) mindestens ein elastisches Element (46, 106) umfasst, welches den Kolben (32) in Richtung seiner flussaufwärts gelagerten Position zurückstellt.

30. Betätigungssystem nach einem der vorherigen Ansprüche, **dadurch gekennzeichnet, dass** die Unterstützungsvorrichtung (5) ein Regelmittel (218) umfasst, das den Wert der Unterstützungskraft (Fₐ) in Abhängigkeit vom flussaufwärts gelagerten Druck Ph in der flussaufwärts gelagerten Kammer (34) des Unterstützungszylinders (30), oder vom flussabwärts gelagerten Druck Ph in der flussabwärts gelagerten Kammer (36), oder von einer Kombination der beiden Drücke entsprechend eines vorbestimmten Unterstützungsgesetzes variiert.

31. Betätigungssystem nach einem der vorherigen Ansprüche, **dadurch gekennzeichnet, dass** die Öffnung (520) ein entlang der Achse der Stange (480) in den Kolben (320) eingebohrter Kanal ist und das Ende dieser Stange (480) eine zur Form des Austritts der Öffnung (520) komplementäre Form hat, um die Versperrung dieser Öffnung zu bewirken, wenn die Stange (480) in Anlage auf dem Koben ist.
